# EUROPEAN PATENT APPLICATION

(11) **EP 4 768 042 A2**
(43) Date of publication of application: **01.07.2026**
(21) Application number: 26165063.4
(22) Date of filing: 18.01.2023
(51) Int. Cl.: A61K 39/00

(54) **NEBULIZATION OF FAB FRAGMENTS**

(30) Priority: 18.01.2022 GB 202200592
(62) Divisional of application: 23701632.4
(71) Applicant: argenx BV, 9052 Zwijnaarde (Ghent) (BE)
(72) Inventor: HEUZÉ-VOURC'H, Nathalie, 37000 Tours (FR); VAN DER WONING, Paul Sebastian, 9052 Gent (BE); PERCIER, Jean-Michel, 9052 Gent (BE)
(74) Representative: Boult Wade Tennant LLP

(57) **Abstract**

The present invention relates to methods of treating a disease or condition associated with the presence or formation of galectin-10 crystals, wherein the method comprises administering to a subject in need thereof a therapeutically effective amount of a pharmaceutical composition comprising a Fab fragment that binds to galectin-10, wherein the composition is administered as an aerosol. Also included within the invention are pharmaceutical compositions comprising a Fab fragment that binds to galectin-10, and nebulizers comprising said pharmaceutical compositions. The pharmaceutical compositions may be useful for treating a disease or condition associated with the presence or formation of galectin-10 crystals, such as asthma or cystic fibrosis.

## Description

### FIELD OF THE INVENTION

The present invention relates to pharmaceutical compositions comprising a Fab fragment that binds to galectin-10, wherein the composition is formulated for aerosolization. The invention also relates to methods of treating a disease or condition associated with the presence or formation of galectin-10 crystals, and pharmaceutical compositions for use in the same methods. The methods comprise administering the pharmaceutical composition as an aerosol, for instance by using a nebulizer. The invention also relates to nebulizers comprising the pharmaceutical compositions.

### BACKGROUND OF THE INVENTION

Asthma is a chronic inflammatory disease of the conducting airways affecting 300 million people worldwide. Pathologically, the disease is characterized by airway eosinophilia and by excessive production of thickened mucus that in some patients can lead to fatal eosinophilic inflammatory responses, such as irreversible obstruction of small airways.

In eosinophilic asthma, eosinophils are very abundant in the airway epithelium and can undergo eosinophil extracellular trap cell death (EETosis). During the process of EETosis, eosinophils release nuclear chromatin together with galectin-10 protein. At high concentration, extracellular galectin-10 starts to crystalize forming distinctive and colourless crystals called Charcot Leyden crystals (CLCs). These CLCs have been found to boost type 2 immunity in asthmatics and contribute to increased viscoelasticity of mucus and anchoring of the mucus plug to the airway epithelium. CLCs have also been observed in human tissues and secretions in association with an eosinophilic inflammatory response such as myeloid leukemia, allergic and parasitic diseases, and eosinophilic cystic fibrosis.

The present inventors have previously shown that anti-galectin-10 antibodies are capable of solubilizing CLCs (WO2019/197675). It is reported how galectin-10 crystals can induce a pro-inflammatory response *in vivo* and how this response can be suppressed by the administration of galectin-10 antibodies capable of disrupting galectin-10 crystallization; Importantly, galectin-10 antibodies were able to dissolve CLCs from patient mucus samples. Taken together, this demonstrates how agents that target galectin-10 crystallization can be used to treat conditions and disorders where the pathology is linked to the presence of CLCs.

The CLCs are present in the mucus of these patients and therefore in the lumen of the airways. Accordingly, the only effective way to reach these CLCs is by local delivery to the airways. Aerosolization is routinely used for the delivery of small drug molecules; however, administration of proteins by inhalation is rare. For monoclonal antibodies (mAb) in particular, there remain many challenges in terms of aerosol technology and drug formulation. Aerosolization imposes physical stresses on mAbs likely to induce changes in protein conformation, potentially decreasing their biological activity and causing them to become immunogenic.

Nebulizers are the most widely used inhalers for generating aerosols from protein solutions; however, nebulizers have not proved completely successful due to observations of lower levels of activity, partial protein degradation and higher levels of aggregation (Respaud et al. 2014; mAbs; 6(5): 1347-1355). These aggregates can cause a loss in therapeutic activity, and as a result, formulations may need to be optimized in an attempt to reduce aggregate formation. Accordingly, there exists a need for further improvements in the delivery of protein therapies by inhalation.

### SUMMARY OF INVENTION

The development of antibody treatments suitable for delivery via inhalation has proved challenging for drug formulators. Aerosolization imposes significant physical stresses on antibodies, often resulting in the formation of aggregates. This can contribute to decreased biological activity and increased immunogenicity caused by anti-drug antibody (ADA) responses. The present inventors have identified that aerosolization of Fab fragments results in the formation of very few, and in many cases no, protein aggregates. In particular, it was found that a Fab fragment derived from a full-length IgG could be successfully aerosolized whereas aerosolization of the full-length IgG counterpart resulted in aggregation. It is reported herein that aerosolized Fab fragments that bind to galectin-10, not only produce few aggregates, but also retain their biological activity and potency. Specifically, these aerosolized Fab fragments retain binding to galectin-10 and retain the ability to solubilise galectin-10 crystals (CLC's). As a result, it is possible to prepare pharmaceutical formulations comprising Fab fragments without the need for added surfactants, or with only low concentrations of added surfactant.

In a first aspect, the present invention provides a pharmaceutical composition comprising a Fab fragment that binds to galectin-10, for use in a method of treating a disease or condition associated with the presence or formation of galectin-10 crystals, wherein the method comprises administering to a subject in need thereof a therapeutically effective amount of the pharmaceutical composition, wherein the composition is administered as an aerosol.

In a second related aspect, the present invention provides a method of treating a disease or condition associated with the presence or formation of galectin-10 crystals, wherein the method comprises administering to a subject in need thereof a therapeutically effective amount of a pharmaceutical composition comprising a Fab fragment that binds to galectin-10, wherein the composition is administered as an aerosol.

In certain embodiments, the disease or condition associated with the presence or formation of galectin-10 crystals is selected from the group consisting of: asthma; chronic rhinosinusitis; celiac disease; helminth infection; gastrointestinal eosinophilic inflammation; cystic fibrosis (CF); allergic bronchopulmonary aspergillosis (ABPA); Churg-Straus vasculitis; chronic eosinophilic pneumonia; and acute myeloid leukemia. In certain preferred embodiments, the disease or condition associated with the presence or formation of galectin-10 crystals is asthma or cystic fibrosis (CF).

In certain embodiments, the Fab fragment inhibits crystallization of galectin-10 when bound to soluble galectin-10, and/or promotes dissolution of crystalline galectin-10 when bound to crystalline galectin-10. In certain embodiments, the crystalline galectin-10 is Charcot-Leyden crystals (CLCs).

In certain embodiments, the Fab fragment binds to human galectin-10. In certain preferred embodiments, the Fab fragment comprises a variable heavy chain (VH) domain and a variable light chain (VL) domain,
wherein the VH domain comprises:
   HCDR3 comprising or consisting of SEQ ID NO: 4 (GLRVGVLGFDY);
   HCDR2 comprising or consisting of SEQ ID NO: 3 (AINAGGGSTSYADSVKG); and
   HCDR1 comprising or consisting of SEQ ID NO: 2 (SYAMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the Fab fragment comprises a variable heavy chain (VH) domain and a variable light chain (VL) domain, wherein:
the VH domain comprises the amino acid sequence of SEQ ID NO: 1 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 1 
(EVQLLESGGGLVQPGGSLRLSCAASGFTFSSYAMSWVRQAPGKGLEWVSAINAGGGSTSYAD
SVKGRFTISRDNSKNTLYLQMNSLRAEDTAVYYCAKGLRVGVLGFDYWGQGTLVTVSS);
and the VL domain comprises the amino acid sequence of SEQ ID NO: 5

(SYELTQPLSVSVALGQTARITCDGNNIGSKSAQWYQQKPGQAPVLVIYADEYRPEGIPERFSGS
NSGNTATLTISRAQAGDEADYYCQVWDGSAAVFGGGTQLTVL).

In certain embodiments, the Fab fragment is humanized, germlined, or deimmunized.

In certain embodiments, the pharmaceutical composition is administered to the subject via inhalation. In certain embodiments, the pharmaceutical composition is administered as an aerosol using a nebulizer, a pressurised metered-dose inhaler (pMDI), a dry powder inhaler, a soft-mist inhaler, or a smart inhaler. In certain preferred embodiments, the pharmaceutical composition is administered as an aerosol using a nebulizer. In certain embodiments, the pharmaceutical composition is administered as an aerosol using a jet nebulizer, an ultrasonic nebulizer, or a mesh nebulizer.

In certain embodiments, prior to administration, the pharmaceutical composition is an aqueous liquid formulation. In certain embodiments, the pharmaceutical composition comprises a pharmaceutically acceptable carrier or excipient. In certain embodiments, the excipient is a liquid excipient, optionally a solvent or an aqueous solvent.

In certain embodiments, the composition comprises a surfactant in an amount less than 5%, less than 2%, less than 1%, less than 0.5%, less than 0.1%, less than 0.05%, less than 0.02% or less than 0.01%. In certain alternative embodiments, the composition does not contain surfactant.

In certain embodiments, the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml. In certain embodiments, the concentration of the Fab fragment in the composition is about 1, 2, 3, 4, 5, 6, 7, 8, 9, 10, 11, 12, 13, 14 or 15 mg/ml.

In certain embodiments, the particle size of particles in the aerosol is less than 30 µm, less than 25 µm, less than 10 µm, less than 2 µm, less than 1 µm, less than 0.5 µm, less than 0.4 µm, less than 0.3 µm, less than 0.2 µm, or less than 0.1 µm. In certain preferred embodiments, the particle size of particles in the aerosol is less than 2 µm.

In certain embodiments, the concentration of particles in the aerosol is less than 2 x10⁵, less than 1 x10⁵, less than 2 x 10⁴, or less than 1 x 10⁴ total particles/ml.

In certain embodiments, the concentration of particles of size greater than 2 µm in the aerosol is less than 1 x10⁵, less than 5 x 10⁴, less than 2 x10⁴, less than 1 x 10⁴, less than 1 x 10³, or less than 1 x 10² particles > 2 µm /ml.

In a third aspect, the present invention provides a pharmaceutical composition comprising a Fab fragment that binds to galectin-10, wherein the composition is formulated for aerosolization.

The embodiments described above relating to the pharmaceutical compositions in the first and second aspects of the invention apply equally to the pharmaceutical composition of the third aspect.

In a fourth aspect, the present invention provides a nebulizer comprising any of the pharmaceutical compositions described herein. In certain embodiments, there is provided a nebulizer comprising a pharmaceutical composition comprising a Fab fragment that binds to galectin-10, wherein the composition is formulated for aerosolization.

### BRIEF DESCRIPTION OF THE DRAWINGS

**Figure 1** shows aerosol droplet size analysis by laser diffraction. The raw data is presented in Figure 1A. Each sample was tested at two concentrations (3 and 10 mg/ml), and aerosolization was performed using two different devices, Aerogen^{®} Solo and Pari eFlow^{®}. Experiments were run in triplicate using three Aerogen^{®} Solo devices and three Pari eFlow^{®} devices, identified by their serial numbers. The bar chart in Figure 1B shows the median range of Volume Mean Diameter (VMD) during nebulization of each sample at the two concentrations (3 and 10 mg/ml).
**Figure 2** shows the protein sample concentration before nebulisation (3 and 10 mg/ml) and after nebulisation using two different devices, Aerogen^{®} Solo and Pari eFlow^{®}. The raw data is presented in Figure 2A. Experiments were run in triplicate using three Aerogen^{®} Solo devices and three Pari eFlow^{®} devices, identified by their serial numbers. The bar chart in Figure 2B shows the median range of concentration variation (%) after nebulization of the tested antibodies/fragments.

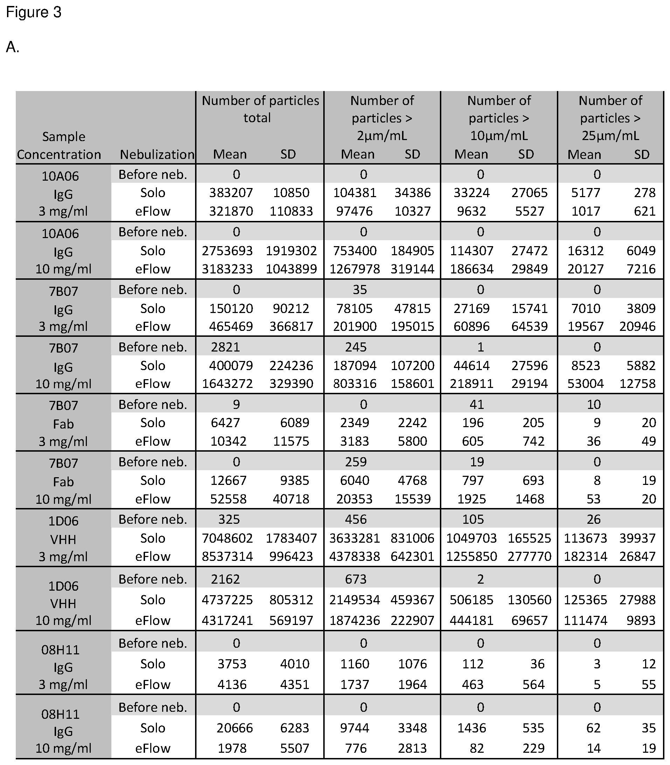

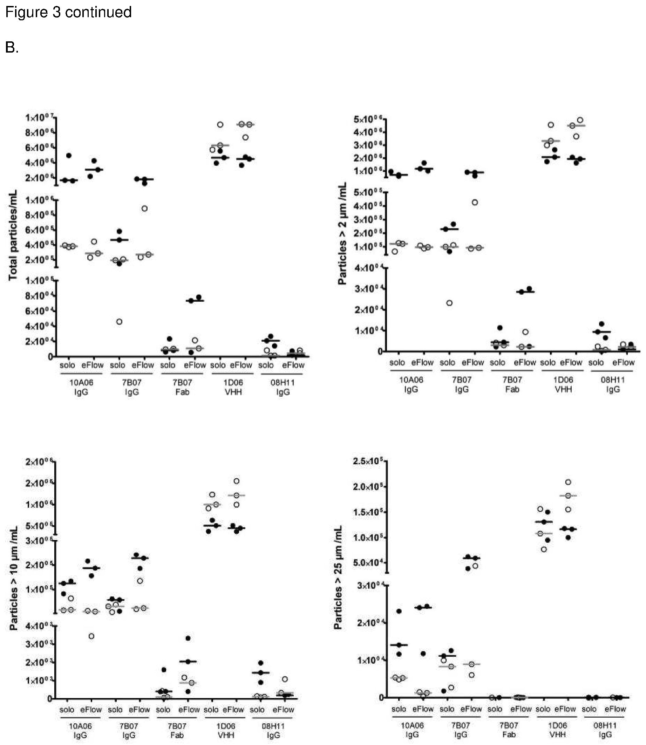

**Figure 3** shows the effect of nebulization on aggregate formation. Aggregates are detected by Flow Cell Microscopy (FCM) and are expressed as the median number of particles/ml after nebulization with the Aerogen^{®} Solo device or Pari eFlow^{®} device. Mean of the number of particles with the buffer only was subtracted from the sample value. The number of particles per mL is expressed as total particles and as particles with an equivalent diameter >2 µm, >10 µm and >25 µm. In Figure 3B, black and white circles represent data post-nebulization for the concentration 10 mg/mL and 3 mg/mL, respectively.
**Figure 4** shows dynamic light scattering (DLS) analysis of samples before and after nebulization. Parameters of the main peak are represented for each sample. If necessary (multimodal), the remaining samples were filtered before a new analysis.
**Figure 5** shows the percentage monomers analysed by SEC-HPLC of samples before and after nebulization. Results in Figure 5A are expressed as the mean mass percentage of monomer and High Molecular Weight (HMW) species. The bar chart in Figure 5B shows the percentage of high molecular weight species. Samples were analyzed before nebulization (1), and after nebulization with: Aerogen^{®} Solo device (2) and Pari eFlow^{®} (3).
**Figure 6** shows the protein concentration of each of the tested Fab clones before and after nebulization with the Aerogen Solo vibrating mesh nebulizer. The experiment was run in triplicate using three different Aerogen Solo devices. Figure 6A and Figure 6B provide two independent experiments.
**Figure 7** provides an overview of dynamic light scattering (DLS) results for all Fab clones before and after mesh nebulization at T0 and after 4 weeks storage at +5°C (4W). All samples were subjected to a prior filtration step (0.2µm).
**Figure 8** shows subvisible particle counts by Flow Cell Microscopy (FCM) for all nebulized Fabs (upper left pane, total particles/mL; upper right pane, >2□m particles/mL; lower left pane, >10□m particles/mL; lower right pane, >25µm particles/mL; circles - pre-nebulization; squares - post-nebulization).
**Figure 9** shows the binding activity assessed by SPR (Biacore 3000) for all tested Fabs after nebulization. All clones were found to be fully functional without loss in percent relative activity towards the unstressed reference material (pre-nebulization) of each clone.
**Figure 10** shows the percent purity assessed by SEC-HPLC for all nebulized Fabs.
**Figure 11** shows the percent purity assessed by capillary gel electrophoresis (cGE) for all nebulized Fabs. Figure 11A illustrates % purity of the intact Fab under non-reducing conditions and Figure 11B illustrates % total Fab purity under reducing conditions.
**Figure 12** shows the potency of the tested Fabs, 18C06, 20H09, 23H09 and 24F02_N53A (and 7B07_N53A) for GAL10 crystal dissolution.
**Figure 13** shows the potency of the tested Fabs, 18C06, 20H09, and 23H09 (and 7B07_N53A), for GAL10 crystal dissolution after nebulization prior to and post 4 weeks storage at +5°C (assay 1).
**Figure 14** shows the potency of the tested Fabs, 23H09 and 24F02_N53A, for GAL10 crystal dissolution after nebulization prior to and post 4 weeks storage at +5°C (assay 2).
**Figure 15** shows global DRB1 risk scores for a set of 44 marketed therapeutic antibodies. Human antibodies are shown by medium grey bars, humanized by light grey bars and chimeric by dark grey bars. Scores are shown for the tested Fabs, 18C06, 20H09, 23H09 and 24F02_N53A.
**Figure 16** shows the percentage of donors with an IFNγ (left pane) and an IL-5 (right pane) response (DFR2x and DFReq stand for the statistical analyses used at Lonza).
**Figure 17** shows IFNγ (upper pane) and IL-5 (lower pane) response ranking of the four tested molecules in the 31 donor test population (DFR2x and DFReq stand for the statistical analyses used at Lonza).
**Figure 18** provides an overview of the tested Fab attributes after nebulization.

### DETAILED DESCRIPTION

### A. Definitions

Unless defined otherwise, all technical and scientific terms used herein have the same meaning as commonly understood by the ordinary person skilled in the art to which the invention pertains. Without limiting any term, further clarifications of some of the terms used herein are provided below.

As used herein, the term **galectin-10 (or Gal10 or Gal-10)** refers to the small, hydrophobic glycan binding protein that autocrystallizes to form Charcot-Leyden crystals. Galectin-10 is also referred to as Charcot-Leyden crystal protein (CLCP), eosinophil lysophospholipase and lysolecithin acylhydrolase. The term "galectin-10" is broad enough to cover the human protein and any species homologues. Gal10 is a member of the Galectin family, characterized by galactose binding domains, from which the different members have been shown to positively or negatively modulate multiple steps of inflammation. This small, auto crystallizing, and hydrophobic protein (only 16.5 kDa) is localized in the cytoplasm of eosinophils. This protein is among the most abundant of eosinophilic constituents, representing 7%-10% of total cellular protein. Importantly, the Gal10 gene is only found in humans and non-human primates. Gal10 lacks a secretion peptide signal and transmembrane domain and is released during eosinophil extracellular trap cell death (EETosis)

The amino acid sequence of the full-length human galectin-10 is represented by SEQ **ID** NO: 9 (see below). This sequence corresponds to the sequence deposited in the UniProt database as human galectin-10, accession number Q05315. Also encompassed within the term "galectin-10" are naturally occurring variants of the human sequence, for example the Ala→Val variant on position 28.

SEQ ID NO: 9

1 10 20 30 40 50
MSLLPVPYTE AASLSTGSTV TIKGRPLACF LNEPYLQVDF HTEMKEESDI
60 70 80 90 100
VFHFQVCFGR RVVMNSREYG AWKQQVESKN MPFQDGQEFE LSISVLPDKY
110 120 130 140
QVMVNGQSSY TFDHRIKPEA VKMVQVWRDI SLTKFNVSYL KR

As used herein, the terms **galectin-10 crystals** or **Charcot-Leyden crystals (CLCs)** are used herein interchangeably to refer to crystals formed of galectin-10. The crystals formed by galectin-10 are typically bi-pyramidal hexagonal crystals and are approximately 20-40 µm in length and approximately 2-4 µm width. These crystals have been associated with eosinophilic inflammatory disorders.

As used herein, the term **antibody** or **immunoglobulin** refers to structures comprising light and heavy chains, with or without an interchain covalent linkage between them. The term "immunoglobulin" includes a polypeptide having a combination of two heavy and two light chains whether or not it possesses any relevant specific immunoreactivity. "Antibodies" refer to such assemblies which have significant known specific immunoreactive activity to an antigen of interest (herein galectin-10). The term "galectin-10 antibodies" is used herein to refer to antibodies which exhibit immunological specificity for the galectin-10 protein, including human galectin-10, and in some cases species homologues thereof.

The generic term "immunoglobulin" comprises five distinct classes of antibody that can be distinguished biochemically. The following discussion will generally be directed to the IgG class of immunoglobulin molecules. With regard to IgG, immunoglobulins comprise two identical light polypeptide chains of molecular weight approximately 23,000 Daltons, and two identical heavy chains of molecular weight 53,000-70,000. The four chains are joined by disulfide bonds in a "Y" configuration wherein the light chains bracket the heavy chains starting at the mouth of the "Y" and continuing through the variable region.

The light chains of an antibody are classified as either kappa or lambda (κ, λ). Each heavy chain class may be bound with either a kappa or lambda light chain. In general, the light and heavy chains are covalently bonded to each other, and the "tail" portions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. Those skilled in the art will appreciate that heavy chains are classified as gamma, mu, alpha, delta, or epsilon, (γ, µ, α, δ, or ε) with some subclasses among them (e.g., γ1-γ4). It is the nature of this chain that determines the "class" of the antibody as IgG, IgM, IgA, IgD or IgE, respectively. The immunoglobulin subclasses (isotypes) e.g., IgG1, IgG2, IgG3, IgG4, IgA1, etc. are well characterized and are known to confer functional specialization. Modified versions of each of these classes and isotypes are readily discernible to the skilled artisan in view of the instant disclosure.

The variable region of an antibody allows the antibody to selectively recognize and specifically bind epitopes on antigens. That is, the VL domain and VH domain of an antibody combine to form the variable region that defines a three dimensional antigen binding site. This quaternary antibody structure forms the antigen binding site present at the end of each arm of the Y. More specifically, the antigen binding site is defined by three complementary determining regions (CDRs) on each of the VH and VL chains.

As used herein, the term **VHH antibody** or **heavy chain-only antibody** refers to a type of antibody produced only by species of the Camelidae family, which includes camels, llama, alpaca. Heavy chain-only antibodies or VHH antibodies are composed of two heavy chains and are devoid of light chains. Each heavy chain has a variable domain at the N-terminus, and these variable domains are referred to as "VHH" domains in order to distinguish them from the variable domains of the heavy chains of the conventional heterotetrameric antibodies i.e. the VH domains, described above.

As used herein, the terms **variable region** or **variable domain** are used herein interchangeably and are intended to have equivalent meaning. The term "variable" refers to the fact that certain portions of the variable domains VH and VL differ extensively in sequence among antibodies and are used in the binding and specificity of each particular antibody for its target antigen. However, the variability is not evenly distributed throughout the variable domains of antibodies. It is concentrated in three segments called "hypervariable loops" in each of the VL domain and the VH domain which form part of the antigen binding site. The first, second and third hypervariable loops of the VLambda light chain domain are referred to herein as L1(λ), L2(λ) and L3(λ) and may be defined as comprising residues 24-33 (L1(λ), consisting of 9, 10 or 11 amino acid residues), 49-53 (L2(λ), consisting of 3 residues) and 90-96 (L3(λ), consisting of 5 residues) in the VL domain (Morea et al., Methods 20:267-279 (2000)). The first, second and third hypervariable loops of the VKappa light chain domain are referred to herein as L1(κ), L2(κ) and L3(κ) and may be defined as comprising residues 25-33 (L1(κ), consisting of 6, 7, 8, 11, 12 or 13 residues), 49-53 (L2(κ), consisting of 3 residues) and 90-97 (L3(κ), consisting of 6 residues) in the VL domain (Morea et al., Methods 20:267-279 (2000)). The first, second and third hypervariable loops of the VH domain are referred to herein as H1, H2 and H3 and may be defined as comprising residues 25-33 (H1, consisting of 7, 8 or 9 residues), 52-56 (H2, consisting of 3 or 4 residues) and 91-105 (H3, highly variable in length) in the VH domain (Morea et al., Methods 20:267-279 (2000)).

Unless otherwise indicated, the terms L1, L2 and L3 respectively refer to the first, second and third hypervariable loops of a VL domain, and encompass hypervariable loops obtained from both Vkappa and Vlambda isotypes. The terms H1, H2 and H3 respectively refer to the first, second and third hypervariable loops of the VH domain, and encompass hypervariable loops obtained from any of the known heavy chain isotypes, including γ, ε, δ, α or µ.

The hypervariable loops L1, L2, L3, H1, H2 and H3 may each comprise part of a "complementarity determining region" or "CDR", as defined below. The terms "hypervariable loop" and "complementarity determining region" are not strictly synonymous, since the hypervariable loops (HVs) are defined on the basis of structure, whereas complementarity determining regions (CDRs) are defined based on sequence variability (Kabat et al., Sequences of Proteins of Immunological Interest, 5th Ed. Public Health Service, National Institutes of Health, Bethesda, MD., 1983) and the limits of the HVs and the CDRs may be different in some VH and VL domains.

The CDRs of the VL and VH domains can typically be defined as comprising the following amino acids: residues 24-34 (LCDR1), 50-56 (LCDR2) and 89-97 (LCDR3) in the light chain variable domain, and residues 31-35 or 31-35b (HCDR1), 50-65 (HCDR2) and 95-102 (HCDR3) in the heavy chain variable domain; (Kabat et al., Sequences of Proteins of Immunological Interest, 5th Ed. Public Health Service, National Institutes of Health, Bethesda, MD. (1991)). Thus, the HVs may be comprised within the corresponding CDRs and references herein to the "hypervariable loops" of VH and VL domains should be interpreted as also encompassing the corresponding CDRs, and vice versa, unless otherwise indicated.

The more highly conserved portions of variable domains are called the framework region (FR), as defined below. The variable domains of native heavy and light chains each comprise four FRs (FR1, FR2, FR3 and FR4, respectively), largely adopting a β-sheet configuration, connected by the three hypervariable loops. The hypervariable loops in each chain are held together in close proximity by the FRs and, with the hypervariable loops from the other chain, contribute to the formation of the antigen binding site of antibodies. Structural analysis of antibodies revealed the relationship between the sequence and the shape of the binding site formed by the complementarity determining regions (Chothia et al., J. Mol. Biol. 227: 799-817 (1992)); Tramontano et al., J. Mol. Biol, 215:175-182 (1990)). Despite their high sequence variability, five of the six loops adopt just a small repertoire of main-chain conformations, called "canonical structures". These conformations are first of all determined by the length of the loops and secondly by the presence of key residues at certain positions in the loops and in the framework regions that determine the conformation through their packing, hydrogen bonding or the ability to assume unusual main-chain conformations.

As used herein, the term **CDR** or **complementarity determining region** means the non-contiguous antigen binding sites found within the variable region of both heavy and light chain polypeptides. These particular regions have been described by Kabat et al., J. Biol. Chem. 252, 6609-6616 (1977) and Kabat et al., Sequences of protein of immunological interest. (1991), and by Chothia et al., J. Mol. Biol. 196:901-917 (1987) and by MacCallum et al., J. Mol. Biol. 262:732-745 (1996) where the definitions include overlapping or subsets of amino acid residues when compared against each other. The amino acid residues which encompass the CDRs as defined by each of the above cited references are set forth for comparison. Preferably, the term "CDR" is a CDR as defined by Kabat based on sequence comparisons.

**Table 1: CDR definitions**

| | CDR Definitions | | |
|---|---|---|---|
| | Kabat¹ | Chothia² | MacCallum³ |
| V_{H} CDR1 | 31-35 | 26-32 | 30-35 |
| V_{H} CDR2 | 50-65 | 53-55 | 47-58 |
| V_{H} CDR3 | 95-102 | 96-101 | 93-101 |
| V_{L} CDR1 | 24-34 | 26-32 | 30-36 |
| V_{L} CDR2 | 50-56 | 50-52 | 46-55 |
| V_{L} CDR3 | 89-97 | 91-96 | 89-96 |

| | | | |
|---|---|---|---|
| ¹Residue numbering follows the nomenclature of Kabat *et al.,* supra ²Residue numbering follows the nomenclature of Chothia *et al.,* supra ³Residue numbering follows the nomenclature of MacCallum *et al.,* supra | | | |

As used herein, the term **framework region** or **FR region** includes the amino acid residues that are part of the variable region, but are not part of the CDRs (e.g., using the Kabat definition of CDRs). Therefore, a variable region framework is between about 100-120 amino acids in length but includes only those amino acids outside of the CDRs. For the specific example of a heavy chain variable domain and for the CDRs as defined by Kabat *et al.,* framework region 1 corresponds to the domain of the variable region encompassing amino acids 1-30; framework region 2 corresponds to the domain of the variable region encompassing amino acids 36-49; framework region 3 corresponds to the domain of the variable region encompassing amino acids 66-94, and framework region 4 corresponds to the domain of the variable region from amino acids 103 to the end of the variable region. The framework regions for the light chain are similarly separated by each of the light chain variable region CDRs. Similarly, using the definition of CDRs by Chothia *et al.* or McCallum *et al.* the framework region boundaries are separated by the respective CDR termini as described above. In preferred embodiments the CDRs are as defined by Kabat.

In naturally occurring antibodies, the six CDRs present on each monomeric antibody are short, non-contiguous sequences of amino acids that are specifically positioned to form the antigen binding site as the antibody assumes its three dimensional configuration in an aqueous environment. The remainder of the heavy and light variable domains show less inter-molecular variability in amino acid sequence and are termed the framework regions. The framework regions largely adopt a β-sheet conformation and the CDRs form loops which connect, and in some cases form part of, the β-sheet structure. Thus, these framework regions act to form a scaffold that provides for positioning the six CDRs in correct orientation by inter-chain, non-covalent interactions. The antigen binding site formed by the positioned CDRs defines a surface complementary to the epitope on the immunoreactive antigen. This complementary surface promotes the non-covalent binding of the antibody to the immunoreactive antigen epitope. The position of CDRs can be readily identified by one of ordinary skill in the art.

As used herein, the term **Fab** or **Fab fragment** refers to a molecule composed of a heavy chain and light chain wherein the light chain consists of the VL domain and the one constant domain (CL, Cκ or Cλ) and the heavy chain consists of the VH domain and the CH1 domain only. A Fab fragment is typically one arm of a Y-shaped immunoglobulin molecule. A Fab fragment can be generated from an immunoglobulin molecule by the action of the enzyme papain. Papain cleaves immunoglobulin molecules in the region of the hinge so as yield two Fab fragments and a separate Fc region. A Fab fragment is not to be construed as a full-length antibody molecule.

As used herein, the term **humanizing substitutions** refers to amino acid substitutions in which the amino acid residue present at a particular position in the VH or VL domain of an antibody or fragment (for example a camelid-derived galectin-10 antibody or fragment) is replaced with an amino acid residue which occurs at an equivalent position in a reference human VH or VL domain. The reference human VH or VL domain may be a VH or VL domain encoded by the human germline. Humanising substitutions may be made in the framework regions and/or the CDRs of the antibodies or fragments, defined herein.

As used herein, the term **humanized variant** refers to a variant antibody which contains one or more "humanising substitutions" compared to a reference antibody, wherein a portion of the reference antibody (e.g. the VH domain and/or the VL domain or parts thereof containing at least one CDR) has an amino acid derived from a non-human species, and the "humanising substitutions" occur within the amino acid sequence derived from a non-human species.

As used herein, the term **germlined variant** refers specifically to "humanised variants" in which the "humanising substitutions" result in replacement of one or more amino acid residues present at a particular position(s) in the VH or VL domain of an antibody or fragment (for example a camelid-derived galectin-10 antibody or fragment) with an amino acid residue which occurs at an equivalent position in a reference human VH or VL domain encoded by the human germline. It is typical that for any given "germlined variant", the replacement amino acid residues substituted into the germlined variant are taken exclusively, or predominantly, from a single human germline-encoded VH or VL domain. The terms "humanised variant" and "germlined variant" are often used interchangeably herein. Introduction of one or more "humanising substitutions" into a camelid-derived (e.g. llama derived) VH or VL domain results in production of a "humanised variant" of the camelid (llama)-derived VH or VL domain. If the amino acid residues substituted in are derived predominantly or exclusively from a single human germ line-encoded VH or VL domain sequence, then the result may be a "human germlined variant" of the camelid (Ilama)-derived VH or VL domain.

As used herein, the term **deimmunized or deimmunization** refers to the identification and subsequent removal of a T-cell epitope in a binding molecule. Typically, though not necessarily this is done in the variable region of an antibody or fragment. Again often, but not always necessary, this is done in a framework region and thus outside the CDR regions. Removal of a T-cell epitope is typically achieved by substituting one or more amino acids encoding the T-cell epitope. The sequence is thereby changed into a sequence different from a T-cell epitope. A deimmunized variable region typically contains 1-5 amino acid substitutions. The substituted amino acids are selected such that the tertiary structure of the variable region is not significantly altered. The substituted amino acid is therefore typically selected from the same group of amino acids (i.e. neutral, charged positive, charged negative, lipophilic). When available in the same group an amino acid is substituted for an amino acid that in a structurally close human antibody is at the same or similar position in the variable region.

Deimmunization of a binding molecule for use in human subjects can typically be achieved by modifying the heavy chain at a maximum of 5 places, the light chain at a maximum of 5 places or both. Deimmunization of a variable region is an established technology and always yields a binding molecule with a decreased probability of inducing an immune response in a human. The number of amino acid substitutions that are required to achieve this result is typically less than 5 in each chain. Often the substitution of 1, 2 or 3 amino acids in each chain is sufficient for obtaining a binding molecule with a decreased probability of inducing an immune response in a human when compared to the unmodified sequence(s). The Fab fragment of the invention is preferably a humanized or deimmunized Fab fragment.

As used herein, the term % **sequence identity thereto** refers to the degree of similarity between two sequences. Percent sequence identity between two amino acid sequences may be determined by comparing these two sequences aligned in an optimum manner and in which the amino acid sequence to be compared can comprise additions or deletions with respect to the reference sequence for an optimum alignment between these two sequences. The percentage of identity is calculated by determining the number of identical positions for which the amino acid residue is identical between the two sequences, dividing this number of identical positions by the total number of positions in the comparison window and multiplying the result obtained by 100 in order to obtain the percentage of identity between these two sequences. For example, it is possible to use the BLAST program, "BLAST 2 sequences" (Tatusova et al, "Blast 2 sequences - a new tool for comparing protein and nucleotide sequences", FEMS Microbiol Lett. 174:247-250) available on the site http://www.ncbi.nlm.nih.gov/ gorf/bl2.html, the parameters used being those given by default (in particular for the parameters "open gap penalty": 5, and "extension gap penalty": 2; the matrix chosen being, for example, the matrix "BLOSUM 62" proposed by the program), the percentage of identity between the two sequences to be compared being calculated directly by the program.

As used herein, the term **aerosolization** refers to the dispersal of a substance (such as a medicine) into an aerosol, wherein an aerosol is a suspension of fine solid particulates or liquid droplets in air or another gas. Therapeutic delivery of aerosols to the lung may be provided via nebulizers, pressurized metered-dose inhalers (pMDI), and other devices (e.g. dry powder inhalers, soft-mist inhalers, and smart inhalers).

As used herein, the term **nebulization** refers to the process of administering medication directly by inhalation with the help of a nebulizer that converts liquid medicine into an aerosol that a patient can inhale through a breathing mask or mouthpiece. A **nebulizer** is a device used to convert liquid into a fine spray of aerosol, by means of oxygen, compressed air, or ultrasonic vibration. There are three main types of nebulizers: jet nebulizers, which use compressed gas to make an aerosol; ultrasonic nebulizers, which use high-frequency vibrations to make an aerosol; and mesh nebulizers (also known as vibrating mesh nebulizers), whereby a vibrating element pushes the liquid through a very fine mesh to make an aerosol.

As used herein, the term **surfactant** refers to substances that absorb to surfaces or interfaces to reduce surface or interfacial tension. These agents aid wetting and dispersion of hydrophobic active pharmaceutical ingredients and they usually act by reducing the interfacial tension between solids and liquids in suspensions. Surfactants are often used to prevent the formation of aggregates in biological formulations. Polysorbates are the most widely used surfactants in the pharmaceutical industry. For example, they are added during the formulation of inhaled corticoids (fluticasone and budesonide) to facilitate the dispersion or dissolution of the drugs during nebulization. However, the use of surfactants as emulsifying agents, solubilizers, suspension stabilizers and as wetting agents in formulations intended for administration to human subjects or to animals can lead to significant changes in the biological activity of the active agent in the formulation. Surfactant molecules incorporated into the formulation can exert their effects in several ways, e.g. by influencing the de-aggregation and dissolution of solid dose forms, by controlling the rate of precipitation of drugs administered in solution form, by increasing membrane permeability and affecting membrane integrity, by alteration of drug metabolizing enzyme activity, and by influencing the binding of the drug to the receptor site.

As used herein, the term **aerosol droplet** refers to the droplets that are produced following aerosolization of a pharmaceutical composition. The size of the aerosol droplets may be measured, for example, by laser diffraction to determine whether the majority of the aerosol droplets produced are within the required respirable size range. Aerosol droplet size is an important characteristic of aerosolizer performance. Droplets that are too large do not reach the lower respiratory tract, whereas droplets that are too small are exhaled. Nebulized solutions, for example, must produce aerosol droplets that have diameters between 1 to 5 µm, which is the optimal aerosol droplet size range to be deposited in the lower respiratory tract. Analytical instruments provide the ability to measure and report the droplet size distribution of the sample. Aerosol droplet size is often expressed in terms of mass median aerodynamic diameter (MMAD). MMAD is defined as the diameter at which 50% of the droplets by mass are larger and 50% are smaller. The size distribution may also be expressed as volume mean diameter (VMD), which is the value of the droplet size that divides the population into two equal halves. The VMD is directly related to the mass median diameter (MMD) by the density of the droplets. The VMD refers to the midpoint droplet size (median), where half of the volume of aerosol is in droplets smaller, and half of the volume is in droplets larger than the median. A VMD (DV0.5) of 50 µm, for example, indicates that half of the volume is in droplet sizes smaller than 50 µm, and half the volume is in droplet sizes larger than 50 µm.

As used herein, the terms **particle size** and **particle concentration** are used to refer to the size and quantity of protein particles or protein aggregates present in a protein-containing solution or aerosol. The particle size and particle concentration may be measured in a liquid sample prior to aerosolization, and may also be measured in a sample that has been aerosolized, collected and then condensed. Ideally, pharmaceutical compositions for use in therapy will contain few or no protein particles or protein aggregates both prior to and after aerosolization. These particles may be visible (i.e. macroscopic) or may be subvisible. Various methods are available to determine both the particle size and particle concentration, including but not limited to dynamic light scattering and flow cell microscopy. Particle concentration may be reported as the total number of particles per ml of sample. Particle concentration may also be reported as the number of particles of a certain size per ml of sample or the number of particles of greater than a certain size per ml of sample.

### B. Fab fragments that bind to galectin-10

The present invention relates to pharmaceutical compositions which comprise a Fab fragment that binds to galectin-10. The present inventors have identified that the Fab fragment is the preferred format for aerosolization. It is reported herein that Fab fragments can be subjected to aerosolization with the production of few or in some cases no aggregates.

Any Fab fragment that binds to galectin-10 is contemplated for use in the present invention. The Fab fragment preferentially binds to human galectin-10. In certain embodiments, the Fab fragment binds to human galectin-10. The amino acid sequence of the full-length human galectin-10 is represented by SEQ ID NO: 9. Accordingly, in certain embodiments, the Fab fragment binds to the amino acid sequence of SEQ ID NO: 9. In certain embodiments, the Fab fragment binds to a naturally occurring variant of human galectin-10. In certain embodiments, the Fab fragment binds to a naturally occurring variant of the amino acid sequence of SEQ ID NO: 9. In certain embodiments, the Fab fragment binds to human galectin-10 and cynomolgus galectin-10.

In certain embodiments, the Fab fragment that binds galectin-10 binds to an epitope comprising one or more amino acids from the crystal packing interfaces of galectin-10, preferably human galectin-10. Said epitope may comprise one or more, two or more, three or more, four or more, five or more amino acids selected from the group consisting of: Ser2, Leu3, Leu4, Tyr8, Thr9, Glu10, Ala11, Ala12, Ser13, Thr16, Thr42, Glu43, Met44, Lys45, Asp49, Ile50, Glu68, Tyr69, Gly70, Ala71, Lys73, Gln74, Gln75, Val76, Glu77, Ser78, Lys79, Asn80, Met81, Leu96, Pro97, Asp98, Lys99, GIn101, Met103, Gly106, GIn107, Ser108, Ser109, Tyr110, Thr111, Asp113, His114, Arg115, Ile116, Lys117, Ala120, Gln125, Thr133, Lys134, Phe135, Asn136, Val137, Ser138, Tyr139, Leu140 and Lys141. In certain embodiments, the Fab fragment binds to an epitope comprising Tyr69 or an epitope comprising an amino acid adjacent to Tyr69. In preferred embodiments, the Fab fragment binds to an epitope comprising Tyr69. Alternatively or in addition, the Fab fragment may bind to an epitope comprising one or more amino acids from the dimerization interface of galectin-10. In such embodiments, the Fab fragment may bind to an epitope comprising one or more amino acids selected from the group consisting of: Pro5, Pro7, Leu27, Ala28, Cys29, Leu31, Asn32, Glu33, Pro34, Tyr35, Gln37, His41, Glu46, Glu47, Gln55, Arg60, Arg61, Arg67, Trp72, Gln75, Trp127, Arg128 and Asp129.The amino acid residues or positions of galectin-10 are defined with reference to the human protein sequence identified herein as SEQ ID NO: 9.

Following aerosolization, the Fab fragment retains the ability to bind to galectin-10. In certain embodiments, the Fab fragment binds to galectin-10 after aerosolization with the same or similar binding affinity as the Fab fragment prior to aerosolization. In certain embodiments, the Fab fragment binds to galectin-10 after aerosolization by 50%, 60%, 70%, 80%, 90%, 95%, or 100% relative to the binding affinity of the Fab fragment prior to aerosolization. In certain embodiments, the Fab fragment binds to galectin-10 after aerosolization by 90% or more relative to the binding affinity of the Fab fragment prior to aerosolization.

The Fab fragment may bind to an epitope of galectin-10 and thereby shield a crystal packing interface of galectin-10. A crystal packing interface of galectin-10 is a surface patch of amino acids that contacts one or more neighbouring galectin-10 molecules in the crystalline lattice. By binding to an epitope of galectin-10 that serves to shield a crystal packing interface of galectin-10, the Fab fragment disrupts the crystallization of galectin-10. It follows, that the Fab fragments may shield a crystal packing interface fully or partially, provided that the Fab fragment disrupts the crystallization of galectin-10. In certain embodiments, the Fab fragment, when bound to crystalline galectin-10, promote dissolution of galectin-10. In certain embodiments, the Fab fragment, when bound to soluble galectin-10, inhibits crystallization of galectin-10. In certain embodiments, the Fab fragment promotes dissolution of crystalline galectin-10 when bound to crystalline galectin-10 and inhibits crystallization of galectin-10 when bound to soluble galectin-10.

The antagonistic properties of the galectin-10 Fab fragments described herein may be measured in accordance with the assays described herein. For example, Fab fragments that bind to galectin-10 may be incubated with soluble galectin-10 under experimental conditions that favour galectin-10 crystallization and the ability of the Fab fragments to inhibit this process may be measured. The inhibitory activity of the Fab fragments that bind to galectin-10 may be measured relative to a control, for example a Fab fragment that does not bind to galectin-10. The inhibitory activity of the Fab fragment that binds to galectin-10 may also be measured relative to a control that is a galectin-10 binding molecule without crystallization inhibitory activity. The Fab fragment that binds to galectin-10 may inhibit crystallization of galectin-10 by 100% relative to control, by 90% relative to control, by 80% relative to control, by 70% relative to control.

Alternatively, the Fab fragment that binds to galectin-10 may be incubated with pre-formed galectin-10 crystals and the ability of the Fab fragments to dissolve the crystals may be measured over a suitable time-course. The galectin-10 crystals may be recombinant crystals formed from recombinant galectin-10 produced *in vitro.* Alternatively, the galectin-10 crystals may be crystals obtained from a patient sample, for example crystals obtained from polyps within the nasal or sinus cavities of a patient. In certain embodiments, the Fab fragments that bind to galectin-10 may be capable of dissolving pre-formed galectin-10 crystals over a period of up to 10 hours, up to 12 hours, up to 14 hours, up to 16 hours, up to 18 hours, up to 20 hours. The Fab fragments that bind to galectin-10 may dissolve the crystals completely i.e. by 100%. Alternatively, the Fab fragments that bind to galectin-10 may dissolve the crystals such that over 50% of the crystals are dissolved, over 60% of the crystals are dissolved, over 70% of the crystals are dissolved, over 80% of the crystals are dissolved, over 90% of the crystals are dissolved over the time-course.

Following aerosolization, the Fab fragment that binds to galectin-10 retains the ability to promote dissolution of crystalline galectin-10 when bound to crystalline galectin-10 and/or inhibit crystallization of galectin-10 when bound to soluble galectin-10. In certain embodiments, the Fab fragment promotes dissolution of crystalline galectin-10 after aerosolization to the same or similar extent as the Fab fragment prior to aerosolization. In certain embodiments, the Fab fragment promotes dissolution of crystalline galectin-10 after aerosolization by 50%, 60%, 70%, 80%, 90%, 95%, or 100% relative to the Fab fragment prior to aerosolization. In certain embodiments, the Fab fragment inhibits crystallization of soluble galectin-10 after aerosolization to the same or similar extent as the Fab fragment prior to aerosolization. In certain embodiments, the Fab fragment inhibits crystallization of soluble galectin-10 after aerosolization by 50%, 60%, 70%, 80%, 90%, 95%, or 100% relative to the Fab fragment prior to aerosolization.

### C. Exemplary Fab fragments that bind to galectin-10

Described herein are exemplary Fab fragments that bind to galectin-10 and which are contemplated for use in the present invention. These Fab fragments serve as preferred Fab fragments for use in accordance with the invention. The exemplary Fab fragments that bind to galectin-10 may be defined exclusively with respect to their structural characteristics, as described below.

A preferred Fab fragment that binds to galectin-10 and which is contemplated for use in the present invention is the 24F02_N53A clone described herein.

Accordingly, in certain embodiments, the Fab fragment comprises a variable heavy chain (VH) domain and a variable light chain (VL) domain,
wherein the VH domain comprises:
   HCDR3 comprising or consisting of SEQ ID NO: 4 (GLRVGVLGFDY);
   HCDR2 comprising or consisting of SEQ ID NO: 3 (AINAGGGSTSYADSVKG); and
   HCDR1 comprising or consisting of SEQ ID NO: 2 (SYAMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the Fab fragment comprises a variable heavy chain (VH) domain and a variable light chain (VL) domain, wherein:
the VH domain comprises the amino acid sequence of SEQ ID NO: 1 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 1 
(EVQLLESGGGLVQPGGSLRLSCAASGFTFSSYAMSWVRQAPGKGLEWVSAINAGGGSTSYAD
SVKGRFTISRDNSKNTLYLQMNSLRAEDTAVYYCAKGLRVGVLGFDYWGQGTLVTVSS);
and the VL domain comprises the amino acid sequence of SEQ ID NO: 5

(SYELTQPLSVSVALGQTARITCDGNNIGSKSAQWYQQKPGQAPVLVIYADEYRPEGIPERFSGS
NSGNTATLTISRAQAGDEADYYCQVWDGSAAVFGGGTQLTVL).

A Fab fragment that binds to galectin-10 and which is contemplated for use in the present invention is the 24F02 clone described herein.

Accordingly, in certain embodiments, the Fab fragment comprises a variable heavy chain (VH) domain and a variable light chain (VL) domain,
wherein the VH domain comprises:
   HCDR3 comprising or consisting of SEQ ID NO: 4 (GLRVGVLGFDY);
   HCDR2 comprising or consisting of SEQ ID NO: 25 (AINNGGGSTSYADSVKG); and
   HCDR1 comprising or consisting of SEQ ID NO: 2 (SYAMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the Fab fragment comprises a variable heavy chain (VH) domain and a variable light chain (VL) domain, wherein:
the VH domain comprises the amino acid sequence of SEQ ID NO: 24 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 24 
(EVQLLESGGGLVQPGGSLRLSCAASGFTFSSYAMSWVRQAPGKGLEWVSAINNGGGSTSYA
DSVKGRFTISRDNSKNTLYLQMNSLRAEDTAVYYCAKGLRVGVLGFDYWGQGTLVTVSS);
and the VL domain comprises the amino acid sequence of SEQ ID NO: 5

(SYELTQPLSVSVALGQTARITCDGNNIGSKSAQWYQQKPGQAPVLVIYADEYRPEGIPERFSGS
NSGNTATLTISRAQAGDEADYYCQVWDGSAAVFGGGTQLTVL).

A Fab fragment that binds to galectin-10 and which is contemplated for use in the present invention is the 18C06 clone described herein.

Accordingly, in certain embodiments, the Fab fragment comprises a variable heavy chain (VH) domain and a variable light chain (VL) domain,
wherein the VH domain comprises:
   HCDR3 comprising or consisting of SEQ ID NO: 13 (GGATVWGRFHDY);
   HCDR2 comprising or consisting of SEQ ID NO: 12 (GINSGDGSSVKG); and
   HCDR1 comprising or consisting of SEQ ID NO: 11 (SFAMS); and
wherein the VL domain comprises:
   LCDR3 comprising or consisting of SEQ ID NO: 17 (GCYDSKLSTYV);
   LCDR2 comprising or consisting of SEQ ID NO: 16 (NANNRAS); and
   LCDR1 comprising or consisting of SEQ ID NO: 15 (TGSSSNIGSGSYLS).

In certain embodiments, the Fab fragment comprises a variable heavy chain (VH) domain and a variable light chain (VL) domain, wherein:
the VH domain comprises the amino acid sequence of SEQ ID NO: 10 or an amino acid sequence having at least 90%, 95%, 97%, 98% or 99% identity thereto, and
the VL domain comprises the amino acid sequence of SEQ ID NO: 14, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 10 
(EVQLLESGGGLVQPGGSLRLSCAASGFTFSSFAMSWVRQAPGKGLEWVSGINSGDGSSVKGRFTI
SRDNSKNTLYLQMNSLRAEDTAVYYCAKGGATVWGRFHDYWGQGTLVTVSS);
and the VL domain comprises the amino acid sequence of SEQ ID NO: 14

(QSVLTQPPSVSGAPGQRVTISCTGSSSNIGSGSYLSWYQQLPGTAPKLLIYNANNRASGVPDRFSG
SKSGTSASLAITGLQAEDEADYYCGCYDSKLSTYVFGGGTKLTVL).

A Fab fragment that binds to galectin-10 and which is contemplated for use in the present invention is the 20H09 clone described herein.

Accordingly, in certain embodiments, the Fab fragment comprises a variable heavy chain (VH) domain and a variable light chain (VL) domain,
wherein the VH domain comprises:
   HCDR3 comprising or consisting of SEQ ID NO: 21 (THGIGTLGFGS)
   HCDR2 comprising or consisting of SEQ ID NO: 20 (AIKWHRITYYADSVKG); and
   HCDR1 comprising or consisting of SEQ ID NO: 19 (SYDMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the Fab fragment comprises a variable heavy chain (VH) domain and a variable light chain (VL) domain, wherein:
the VH domain comprises the amino acid sequence of SEQ ID NO: 18 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 18 
(EVQLLESGGGLVQPGGSLRLSCAASGFTFRSYDMSWVRQAPGKGLEWVSAIKWHRITYYADSVKG
RFTISRDNSKNTLYLQMNSLRAEDTAVYYCATTHGIGTLGFGSWGQGTLVTVSS);
and the VL domain comprises the amino acid sequence of SEQ ID NO: 5

(SYELTQPLSVSVALGQTARITCDGNNIGSKSAQWYQQKPGQAPVLVIYADEYRPEGIPERFSGSNSG
NTATLTISRAQAGDEADYYCQVWDGSAAVFGGGTQLTVL).

A Fab fragment that binds to galectin-10 and which is contemplated for use in the present invention is the 23H09 clone described herein.

Accordingly, in certain embodiments, the Fab fragment comprises a variable heavy chain (VH) domain and a variable light chain (VL) domain,
wherein the VH domain comprises:
   HCDR3 comprising or consisting of SEQ ID NO: 21 (THGIGTLGFGS);
   HCDR2 comprising or consisting of SEQ ID NO: 23 (AIAQFQHWTYYADSVKG); and
   HCDR1 comprising or consisting of SEQ ID NO: 19 (SYDMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the Fab fragment comprises a variable heavy chain (VH) domain and a variable light chain (VL) domain, wherein:
the VH domain comprises the amino acid sequence of SEQ ID NO: 22 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the Fab fragment comprises a variable heavy chain (VH) domain and a variable light chain (VL), wherein:
the VH domain comprises the amino acid sequence of SEQ ID NO: 22 
(EVQLLESGGGLVQPGGSLRLSCAASGFTFRSYDMSWVRQAPGKGLEWVSAIAQFQHWTYYADSV
KGRFTISRDNSKNTLYLQMNSLRAEDTAVYYCATTHGIGTLGFGSWGQGTLVTVSS);
and the VL domain comprises the amino acid sequence of SEQ ID NO:

(SYELTQPLSVSVALGQTARITCDGNNIGSKSAQWYQQKPGQAPVLVIYADEYRPEGIPERFSGSNSG
NTATLTISRAQAGDEADYYCQVWDGSAAVFGGGTQLTVL).

For embodiments wherein the domains of the Fab fragments are defined by a particular percentage sequence identity to a reference sequence, the VH and/or VL domains may retain identical CDR sequences to those present in the reference sequence such that the variation is present only within the framework regions.

In certain embodiments, the galectin-10 Fab fragments described herein may be camelid-derived. For example, the galectin-10 Fab fragments may be selected from immune libraries obtained by a method comprising the step of immunizing a camelid with the target of interest i.e. galectin-10. The camelid may be immunized with the target protein or polypeptide fragment thereof, or with an mRNA molecule or cDNA molecule expressing the protein or a polypeptide fragment thereof. Methods for producing antibodies or fragments in camelid species and selecting antibodies or fragments against preferred targets from camelid immune libraries are described in, for example, International patent application no. WO2010/001251, incorporated herein by reference.

In certain embodiments, the galectin-10 Fab fragments may be camelid-derived in that they comprise at least one hypervariable (HV) loop or complementarity determining region obtained from a VH domain or a VL domain of a species in the family Camelidae. In particular, the galectin-10 Fab fragments may comprise VH and/or VL domains, or CDRs thereof, obtained by active immunisation of outbred camelids, e.g. llamas, with galectin-10.

The term "obtained from" in this context implies a structural relationship, in the sense that the HVs or CDRs of the Fab fragments embody an amino acid sequence (or minor variants thereof) which was originally encoded by a Camelidae immunoglobulin gene. However, this does not necessarily imply a particular relationship in terms of the production process used to prepare the Fab fragments.

Camelid-derived Fab fragments may be derived from any camelid species, including *inter alia,* llama, dromedary, alpaca, vicuna, guanaco or camel.

Fab fragments comprising camelid-derived VH and VL domains, or CDRs thereof, are typically recombinantly expressed polypeptides, and may be chimeric polypeptides. The term "chimeric polypeptide" refers to an artificial (non-naturally occurring) polypeptide which is created by juxtaposition of two or more peptide fragments which do not otherwise occur contiguously. Included within this definition are "species" chimeric polypeptides created by juxtaposition of peptide fragments encoded by two or more species, e.g. camelid and human.

In certain embodiments, the entire VH domain and/or the entire VL domain may be obtained from a species in the family Camelidae. The camelid-derived VH domain and/or the camelid-derived VL domain may then be subject to protein engineering, in which one or more amino acid substitutions, insertions or deletions are introduced into the camelid amino acid sequence. These engineered changes preferably include amino acid substitutions relative to the camelid sequence. Such changes include "humanisation" or "germlining" wherein one or more amino acid residues in a camelid-encoded VH or VL domain are replaced with equivalent residues from a homologous human-encoded VH or VL domain.

Isolated camelid VH and VL domains obtained by active immunisation of a camelid (e.g. llama) with galectin-10 can be used as a basis for engineering galectin-10 Fab fragments in accordance with the present invention. Starting from intact camelid VH and VL domains, it is possible to engineer one or more amino acid substitutions, insertions or deletions which depart from the starting camelid sequence. In certain embodiments, such substitutions, insertions or deletions may be present in the framework regions of the VH domain and/or the VL domain.

In other embodiments, there are provided "chimeric" molecules comprising camelid-derived VH and VL domains (or engineered variants thereof) and one or more constant domains from a non-camelid antibody, for example human-encoded constant domains (or engineered variants thereof). In such embodiments it is preferred that both the VH domain and the VL domain are obtained from the same species of camelid, for example both VH and VL may be from *Llama glama* or both VH and VL may be from *Llama pacos* (prior to introduction of engineered amino acid sequence variation). In such embodiments both the VH and the VL domain may be derived from a single animal, particularly a single animal which has been actively immunised with the antigen of interest.

As an alternative to engineering changes in the primary amino acid sequence of Camelidae VH and/or VL domains, individual camelid-derived hypervariable loops or CDRs, or combinations thereof, can be isolated from camelid VH/VL domains and transferred to an alternative (i.e. non-Camelidae) framework, e.g. a human VH/VL framework, by CDR grafting.

In non-limiting embodiments, the exemplary galectin-10 Fab fragments having the CDR, VH and/or VL sequences described herein may comprise CH1 domains and/or CL domains (from the heavy chain and light chain, respectively), the amino acid sequence of which is fully or substantially human. For molecules intended for human therapeutic use, it is typical for the entire constant region, or at least a part thereof, to have fully or substantially human amino acid sequence. Therefore, one or both of the CH1 domain and CL domain of the Fab fragments described herein may be fully or substantially human with respect to its amino acid sequence. In the context of the constant region of a humanised Fab fragment, the term "substantially human" refers to an amino acid sequence identity of at least 90%, or at least 92%, or at least 95%, or at least 97%, or at least 99% with a human constant region.

### D. Therapeutic applications

The Fab fragments described herein are capable of promoting dissolution of crystalline galectin-10 and/or inhibiting crystallization of soluble galectin-10, and are therefore useful in methods of preventing and treating diseases and conditions wherein the pathology is linked to the formation/presence of Charcot-Leyden crystals (CLCs). When used as medicaments, the Fab fragments that bind to galenctin-10 are typically formulated as pharmaceutical compositions. Accordingly, the pharmaceutical compositions of the invention, which comprise a Fab fragment that binds to galectin 10, may be used in a method of treating a disease or condition associated with the presence or formation of galectin-10 crystals.

In a first aspect of the invention, there is provided a pharmaceutical composition comprising a Fab fragment that binds to galectin-10, for use in a method of treating a disease or condition associated with the presence or formation of galectin-10 crystals, wherein the method comprises administering to a subject in need thereof a therapeutically effective amount of the pharmaceutical composition, wherein the composition is administered as an aerosol.

In a second related aspect of the invention, there is provided a method of treating a disease or condition associated with the presence or formation of galectin-10 crystals, wherein the method comprises administering to a subject in need thereof a therapeutically effective amount of a pharmaceutical composition comprising a Fab fragment that binds to galectin-10, wherein the composition is administered as an aerosol.

Importantly, all embodiments described in relation to therapeutic applications are equally applicable to both the first and second aspects of the invention.

In preferred embodiments, the pharmaceutical composition comprising a Fab fragment that binds to galectin-10 is a pharmaceutical composition as described elsewhere herein.

The disease or condition to be treated may be any disease or condition associated with the presence or formation of galectin-10 crystals. The disease or condition to be treated may be any disease or condition associated with the presence or formation of CLC's.

In certain embodiments, the subject in need thereof may be a patient that has been diagnosed with a disease or condition associated with the presence or formation of galectin-10 crystals. In other embodiments, the subject in need thereof may be a patient identified as "at risk" of developing a disease or condition associated with the presence or formation of galectin-10 crystals. For patients having diseases or conditions characterised by the presence of galectin-10 crystals, the methods of treatment will typically involve the administration of a pharmaceutical composition comprising a Fab fragment that binds to galectin-10, and which is capable of dissolving the galectin-10 crystals located in the patient's tissues. For patients identified as "at risk" of developing a disease or condition characterised by the formation of galectin-10 crystals, the methods of prevention may involve the administration of a pharmaceutical composition comprising a Fab fragment that binds to galectin-10, and which is capable of inhibiting the crystallization of galectin-10.

Galectin-10 crystals or CLCs have been observed in patients having a range of diseases and conditions. In certain non-limiting embodiments, the disease or condition associated with the presence or formation of galectin-10 crystals may be selected from the group consisting of: asthma; chronic rhinosinusitis; celiac disease; helminth infection; gastrointestinal eosinophilic inflammation; cystic fibrosis (CF); allergic bronchopulmonary aspergillosis (ABPA); Churg-Straus vasculitis; chronic eosinophilic pneumonia; and acute myeloid leukemia. **In** particualrly preferred embodiments, the disease or condition associated with the presence or formation of galectin-10 crystals is asthma or cystic fibrosis (CF).

As noted above, galectin-10 crystals or CLCs are particularly associated with diseases or conditions characterised by eosinophilic inflammation. In preferred embodiments, the pharmaceutical compositions described herein, are used to treat disorders or conditions associated with eosinophilic inflammation.

As used herein, a method of "treating" a disease or condition means curing a disease or condition and/or alleviating or eradicating the symptoms associated with the disease or condition such that the patient's suffering is reduced.

As used herein, the term "therapeutically effective amount" is intended to mean the quantity or dose of Fab fragment present in the pharmaceutical composition that is sufficient to produce a therapeutic effect, for example, the quantity or dose of Fab fragment required to eradicate or at least alleviate the symptoms associated with a disease or condition. An appropriate amount or dose can be determined by a physician, as appropriate. For example, the dose can be adjusted based on factors such as the size or weight of a subject to be treated, the age of the subject to be treated, the general physical condition of the subject to be treated, the condition to be treated, and the route of administration.

For clinical use, in certain embodiments, the pharmaceutical composition is administered to the subject in need thereof as one or more doses where the concentration of Fab fragment is about 0.1 mg/kg body weight to about 20 mg/kg body weight. In certain embodiments, the pharmaceutical composition is administered to the subject in need thereof as one or more doses where the concentration of Fab fragment is about 0.1 mg/kg body weight to about 10 mg/kg body weight.. In certain embodiments, the pharmaceutical composition is administered to the subject in need thereof as one or more doses where the concentration of Fab fragment is about 0.5 mg/kg body weight to about 10 mg/kg body weight.. In certain embodiments, the pharmaceutical composition is administered to the subject in need thereof as one or more doses where the concentration of Fab fragment is about 1 mg/kg body weight to about 10 mg/kg body weight.

### E. Pharmaceutical formulation

When used as medicaments, the Fab fragments that bind to galectin-10 are typically formulated as pharmaceutical compositions. The scope of the invention thus includes pharmaceutical compositions comprising a Fab fragment that binds to galectin-10.

In a first aspect of the invention, as already described elsewhere herein, there is provided a pharmaceutical composition comprising a Fab fragment that binds to galectin-10, for use in a method of treating a disease or condition associated with the presence or formation of galectin-10 crystals, wherein the method comprises administering to a subject in need thereof a therapeutically effective amount of the pharmaceutical composition, wherein the composition is administered as an aerosol.

In a related third aspect, there is provided a pharmaceutical composition comprising a Fab fragment that binds to galectin-10, wherein the composition is formulated for aerosolization.

Importantly, all embodiments described in relation to pharmaceutical formulations are equally applicable to both the first and third aspects of the invention.

As used herein, the term "formulated for aerosolization" means that the pharmaceutical composition is suitable for aerosolization, for example by nebulization. The active ingredient present in the pharmaceutical composition, for example a Fab fragment that binds to galectin-10, must be pharmacologically active following aerosolization, must be delivered to the lungs, to the appropriate site of action, and must remain there until the desired pharmacological effect occurs, whilst avoiding the clearance mechanism of the lungs. The pharmaceutical composition that is formulated for aerosolization may have a physiological pH and osmolarity to avoid irritation, coughing, and bronchoconstriction of the lungs upon inhalation. The physical properties of drug formulations may have an effect on aerosolization rates and aerosol droplet size. The pharmaceutical composition may have a viscosity, ionic strength and/or surface tension that is particularly suited for aerosolization. For instance, the pharmaceutical composition may contain viscosity-enhancing agents and/or may contain electrolytes to reduce the effects of surface charge leading to smaller atomised droplet size for efficient targeting to the lungs. The pharmaceutical composition may contain agents to protect the active protein against proteolysis in the lungs.

In certain embodiments, prior to aerosolization, the pharmaceutical composition is an aqueous liquid formulation. In certain preferred embodiments, prior to aerosolization, the pharmaceutical composition is an aqueous liquid formulation comprising phosphate buffered saline. In certain embodiments, prior to aerosolization, the pharmaceutical composition is a non-aqueous liquid formulation. In certain embodiments, prior to aerosolization, the pharmaceutical composition is a suspension. In certain embodiments, prior to aerosolization, the pharmaceutical composition is a powder.

The pharmaceutical composition may contain a surfactant. Surfactants aid wetting and dispersion of hydrophobic active pharmaceutical ingredients and they usually act by reducing the interfacial tension between solids and liquids in suspensions. Surfactants are often used to prevent the formation of aggregates in biological formulations. In certain embodiments, the pharmaceutical composition comprises one or more surfactants. In certain embodiments, the pharmaceutical composition comprises one or more surfactants at a concentration between 0.001% and 0.1% w/v. In certain embodiments, the pharmaceutical composition comprises one or more surfactants at a concentration of about 0.02% w/v. In certain embodiments, the pharmaceutical composition comprises one or more surfactants at a concentration of less than 0.05% w/v. The skilled person is aware of commercially available surfactants for use in pharmaceutical formulations, including but not limited to polysorbates, sorbitan esters, oleic acid, soy lecithin, sodium lauryl sulfate, docusate sodium, phosphatidylcholine, polyoxyethylene 15 hydroxy stearate, polyoxyethylene alkyl ethers, Brij-35^{®}, Tween-20, Tween-80, polysorbate 20, and polysorbate 80. In certain embodiments, the pharmaceutical composition comprises polysorbate 20. In certain embodiments, the pharmaceutical composition comprises less than 0.05% polysorbate 20. In certain embodiments, the pharmaceutical composition comprises about 0.02% polysorbate 20. In certain embodiments, the pharmaceutical composition comprises Tween 80. In certain embodiments, the pharmaceutical composition comprises less than 0.05% Tween 80. In certain embodiments, the pharmaceutical composition comprises about 0.02% Tween 80.

As demonstrated in the Examples herein, when aerosolized, the pharmaceutical compositions of the invention produce few protein particles and are thus shown to be less sensitive to aggregation. As a result, the pharmaceutical compositions of the invention may not require addition of surfactant, or may require addition of only small amounts of surfactant. This may be particularly beneficial since evidence suggests that the presence of surfactants can negatively affect the molecular integrity of antibodies in therapeutic formulations (Maillet et al. 2007; Pharmaceutical Research; 25(6): 1318-1326).

Accordingly, in certain embodiments, the pharmaceutical composition does not contain surfactant. In other embodiments, the composition comprises a surfactant in an amount less than 0.05%, less than 0.01%, less than 0.002% or less than 0.001% w/v.

In certain embodiments, the pharmaceutical composition comprises a Fab fragment. In certain preferred embodiments, the pharmaceutical composition comprises a Fab fragment that binds galectin-10. The pharmaceutical composition may comprise any of the Fab fragments described herein. It is particularly preferred that the pharmaceutical composition comprises the Fab fragment described herein as 24F02_N53A.

Accordingly, in certain preferred embodiments, the pharmaceutical composition comprises a Fab fragment comprising a variable heavy chain (VH) domain and a variable light chain (VL) domain,
wherein the VH domain comprises:
   HCDR3 comprising or consisting of SEQ ID NO: 4 (GLRVGVLGFDY);
   HCDR2 comprising or consisting of SEQ ID NO: 3 (AINAGGGSTSYADSVKG); and
   HCDR1 comprising or consisting of SEQ ID NO: 2 (SYAMS); and
wherein the VL domain comprises:
   LCDR3 comprising or consisting of SEQ ID NO: 8 (QVWDGSAAV);
   LCDR2 comprising or consisting of SEQ ID NO: 7 (ADEYRPE); and
   LCDR1 comprising or consisting of SEQ ID NO: 6 (DGNNIGSKSAQ).

In certain embodiments, the pharmaceutical composition comprises a Fab fragment comprising a variable heavy chain (VH) domain and a variable light chain (VL) domain, wherein:
the VH domain comprises the amino acid sequence of SEQ ID NO: 1 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

In certain embodiments, the pharmaceutical composition comprises a Fab fragment comprising a variable heavy chain (VH) domain and a variable light chain (VL), wherein:
the VH domain comprises the amino acid sequence of SEQ ID NO: 1 
(EVQLLESGGGLVQPGGSLRLSCAASGFTFSSYAMSWVRQAPGKGLEWVSAINAGGGSTSYAD
SVKGRFTISRDNSKNTLYLQMNSLRAEDTAVYYCAKGLRVGVLGFDYWGQGTLVTVSS);
and the VL domain comprises the amino acid sequence of SEQ ID NO: 5

(SYELTQPLSVSVALGQTARITCDGNNIGSKSAQWYQQKPGQAPVLVIYADEYRPEGIPERFSGS
NSGNTATLTISRAQAGDEADYYCQVWDGSAAVFGGGTQLTVL).

The Fab fragment must be present in the pharmaceutical composition at a suitable concentration to be pharmacologically effective. Too high drug concentrations can cause foaming in some nebulization devices making aerosolization inefficient or even impossible. Therefore, the concentration of Fab fragment should be sufficient to limit aggregation of the protein both before and after aerosolization, and to prevent foaming or bubbling of the composition in the aerosolization device.

In certain embodiments of the pharmaceutical composition, the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml.

In certain embodiments of the pharmaceutical composition, the concentration of the Fab fragment in the composition is about 1, 2, 3, 4, 5, 6, 7, 8, 9, 10, 11, 12, 13, 14, 15, 16, 17, 18, 19 or 20 mg/ml.

In certain preferred embodiments of the pharmaceutical composition, the concentration of the Fab fragment in the composition is about 3 mg/ml. In certain preferred embodiments of the pharmaceutical composition, the concentration of the Fab fragment in the composition is about 10 mg/ml.

The scope of the invention includes pharmaceutical compositions, containing a Fab fragment that binds to galectin-10 as described herein, formulated with one or more pharmaceutically acceptable carriers or excipients. Accordingly, in certain embodiments, the pharmaceutical composition comprises a pharmaceutically acceptable carrier or excipient. In certain embodiments, the excipient is a liquid excipient, optionally a solvent or an aqueous solvent.

Pharmaceutically acceptable excipients that may be used to formulate the compositions include, but are not limited to: ion exchangers, alumina, aluminium stearate, lecithin, serum proteins such as human serum albumin, buffer substances such as phosphates, sodium citrate, glycine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances such as sodium carboxymethylcellulose, polyethylene glycol, polyacrylates, waxes, polyethylene-polyoxypropylene-block polymers, wool fat, glycerol, solubilizing agents such as ethanol, cyclodextrin, stabilizers such as polysorbate 20, polysorbate 80, PVP K30, Solutol^{®}, and viscosity-enhancers such as sucrose.

The Examples described herein utilise a basic formulation of either 3 mg/ml or 10 mg/ml Fab fragment in phosphate buffered saline (Dulbecco's) with 0.02% polysorbate 20 or Tween 80. Accordingly, in certain embodiments, the pharmaceutical composition formulated for aerosolization comprises a Fab fragment and a surfactant such as polysorbate 20. In certain embodiments, the pharmaceutical composition formulated for aerosolization comprises a Fab fragment and a surfactant such as Tween 80. In certain embodiments, the pharmaceutical composition formulated for aerosolization comprises a Fab fragment and a surfactant such as polysorbate 20 in phosphate buffered saline. In certain embodiments, the pharmaceutical composition formulated for aerosolization comprises a Fab fragment and a surfactant such as Tween 80 in phosphate buffered saline. In a specific embodiment, the pharmaceutical composition formulated for aerosolization comprises a Fab fragment between 1-10 mg/ml and a surfactant such as polysorbate 20 at a concentration of between 0.01-0.05%. In a specific embodiment, the pharmaceutical composition formulated for aerosolization comprises a Fab fragment between 1-10 mg/ml and a surfactant such as Tween 80 at a concentration of between 0.01-0.05%. In a specific embodiment, the pharmaceutical composition formulated for aerosolization comprises a Fab fragment between 1-10 mg/ml and a surfactant such as polysorbate 20 at a concentration of between 0.01-0.05% in phosphate buffered saline. In a specific embodiment, the pharmaceutical composition formulated for aerosolization comprises a Fab fragment between 1-10 mg/ml and a surfactant such as Tween 80 at a concentration of between 0.01-0.05% in phosphate buffered saline.

### F. Route of administration

The pharmaceutical compositions of the invention are for delivery as aerosols to the conducting airways. The conducting airways include the nose, pharynx, larynx, trachea, bronchi, and bronchioles. These structures form a continuous passageway for air to move in and out of the lungs. The therapeutic effect of aerosolized therapies is dependent upon the dose of drug deposited and its distribution within the lung. A variety of inhaler devices are available to deliver inhaled drugs. Most inhaled drugs are delivered by pressurized metered dose inhalers (pMDI), dry powder inhalers or nebulizers. However, other devices exist such as soft mist inhalers and smart inhalers.

Metered dose inhalers (MDIs) are the mainstay of asthma and COPD therapy worldwide. MDIs are pocket-sized hand-held drug delivery devices utilizing the energy of compressed propellant for the aerosol generation. They have the drug in solution or suspension in the propellant, or a mixture of propellant and co-solvent. MDIs deliver small metered drug doses either directly to the patients, or via add-on devices, like spacers or Valved Holding Chambers (VHCs). In certain embodiments, the pharmaceutical composition is administered as an aerosol using a metered-dose inhaler, such as a pressurised metered-dose inhaler (pMDI).

A dry-powder inhaler (DPI) is a device that delivers medication to the lungs in the form of a dry powder. DPIs are commonly used to treat respiratory diseases such as asthma, bronchitis, emphysema and COPD. Aerosols of dry powder are created by directing air through an aliquot of loose powder. Because DPIs are breath-actuated, the need to synchronize inhalation with actuation is eliminated. However, the dispersion of the powder into respirable particulates is dependent on the creation of turbulent flow in the inhaler. Most powder-dispensing systems require the use of a carrier substance. This vehicle substance is mixed with the drug to enable the powder to more readily pass out of the device. The carriers that are used typically include lactose and glucose. In certain embodiments whereby the pharmaceutical composition is in powder form, the pharmaceutical composition is administered as an aerosol using a dry powder inhaler (DPI).

A nebulizer changes medication from a liquid to a mist so you can inhale it into your lungs. Nebulizers are made up of a base that holds an air compressor, a small container for liquid medication, and a tube that connects the air compressor to the medication container. Above the medication container is a mouthpiece or mask used to inhale the mist. There are three main types of nebulizers: jet nebulizers, which use compressed gas to make an aerosol; ultrasonic nebulizers, which make an aerosol through high-frequency vibrations; and mesh nebulizers, where liquid passes through a very fine mesh to form the aerosol. In certain embodiments, the pharmaceutical composition is administered as an aerosol using a nebulizer. In certain embodiments, the pharmaceutical composition is administered as an aerosol using a jet nebulizer. In certain embodiments, the pharmaceutical composition is administered as an aerosol using an ultrasonic nebulizer. In certain embodiments, the pharmaceutical composition is administered as an aerosol using a mesh nebulizer.

In accordance with a fourth aspect of the invention, there is provided a nebulizer comprising a pharmaceutical composition as described elsewhere herein. In certain embodiments, there is provided a nebulizer comprising a pharmaceutical composition comprising a Fab fragment that binds to galectin-10, wherein the composition is formulated for aerosolization.

Each aerosolization device generates its drug aerosol differently, for example producing different aerosol droplet sizes, drug output, total lung deposition and distribution. It may therefore be necessary to adapt the pharmaceutical composition to the performance of the specific inhalation device used.

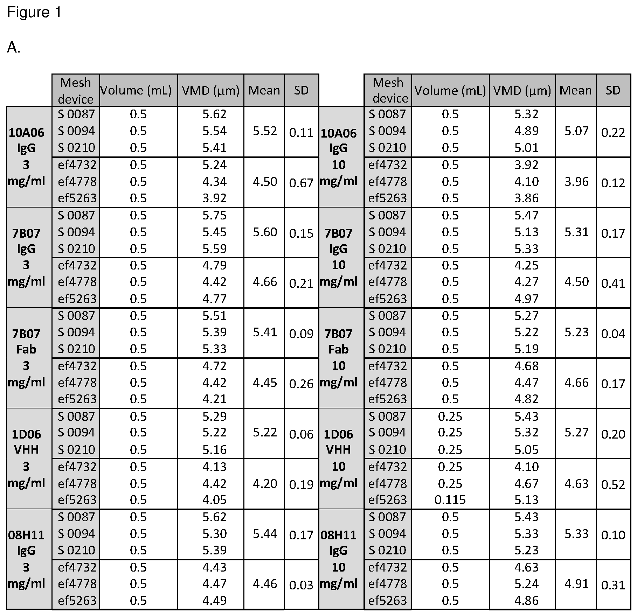

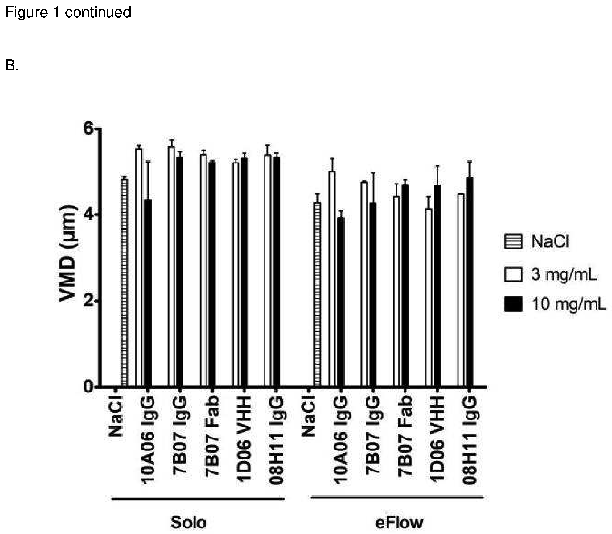

Aerosol droplets produced during aerosolization must be in the size range required for lung deposition. Typically, aerosol droplet sizes of between 1-5 µm are required for deposition in the smaller airways and alveoli. Moreover, aerosol droplets should be as close as possible to monodispersity to increase deposition at the desired site of action and to increase efficacy of treatment. Aerosol droplet size may be measured using laser diffraction, and is typically reported as either volume mean diameter (VMD) or mass median aerodynamic diameter (MMAD). As shown in Example 1 (Figure 1), aerosolization of the pharmaceutical compositions described herein generated aerosol droplets within the size range required for lung deposition. In particular, the VMD after nebulization of the antibody formulations with the Aerogen^{®} Solo device was 4.8 µm, and the VMD after nebulization with the Pari eFlow^{®} device was 4.3 µm.

The process of aerosolization exposes the proteins in the pharmaceutical compositions to physical stresses such as shearing forces and heat, as well as the large air-liquid interface that can alter protein conformation and/or structure through denaturation, chemical modifications (oxidation, deamidation), and aggregation. Device-specific limitations such as the shear forces generated by jet nebulisers, and the temperature increases that occur in ultrasonic nebulisers, can also lead to protein degradation and an increase in undesirable aggregates. The presence of protein aggregates can significantly impact on product quality in terms of biological activity and immunogenicity. Adverse immune reactions, such as anti-drug antibody (ADA) responses, can lead to clinical consequences, such as anaphylaxis, reduced drug half-life, and neutralization of the therapeutic protein. It is thus important to be able to monitor the level of protein aggregates following aerosolization and to limit their numbers.

Protein aggregates can range in size from microscopic to macroscopic. These subvisible and visible particles may be detected in protein-containing solutions using a variety of accepted industry techniques. Biochemical assays for monitoring protein aggregates often rely on ultracentrifugation, size-exclusion chromatography, gel electrophoresis, dynamic light scattering, flow cell microscopy or turbidity measurements. As described in the Examples herein, the pharmaceutical compositions may be nebulized, and the aerosol droplets collected and allowed to condense, so that particle analysis may be performed on the nebulized solution. In the Examples herein, particle size and concentration is determined using a combination of dynamic light scattering and flow cell microscopy; however, it is understood that other well known techniques may be employed to detect and quantify protein aggregates following aerosolization. Importantly, the number of visible and subvisible particles should be as low as possible. In particular, the number of particles of greater than 2 µm in diameter should be as low as possible, since particles of this size are often associated with immunogenicity and severe ADA responses.

Accordingly, in certain embodiments, the particle size of particles in the aerosol is less than 30 µm, less than 25 µm, less than 10 µm, less than 2 µm, less than 1 µm, less than 0.5 µm, less than 0.4 µm, less than 0.3 µm, less than 0.2 µm, or less than 0.1 µm. Preferably, the particle size of particles in the aerosol is less than 2 µm.

Particle size in this instance may refer to the size of all of the particles (protein aggregates) present in the aerosolized sample or may refer to the size of the majority of particles (protein aggregates) present in the aerosolized sample. For example, an aerosolized composition having a particle size of less than 2 µm may mean that all of the particles in the aerosol have a diameter of less than 2 µm. Alternatively, an aerosolized composition having a particle size of less than 2 µm may mean that a majority of the particles in the aerosol have a diameter of less than 2 µm, for instance 60%, 65% 70%, 75%, 80%, 85%, 90%, 95%, or 98% of all the particles in the aerosol have a diameter of less than 2 µm.

The concentration of particles (aggregates) may be quantified as the total number of particles per ml. Preferably, the total number of particles produced following aerosolization will be less than 1 x 10⁵ particles per ml. The concentration of particles may also be quantified as the number of particles of a certain size, for instance, the number of particles of size greater than 2 µm per ml, or greater than 10 µm/ml, or greater than 25 µm/ml.

Accordingly, in certain embodiments, the concentration of particles in the aerosol is less than 2 x10⁵, less than 1 x10⁵, less than 2 x 10⁴, or less than 1 x 10⁴ total particles/ml.

In certain embodiments, the concentration of particles of size greater than 2 µm in the aerosol is less than 1 x10⁵, less than 5 x 10⁴, less than 2 x10⁴, less than 1 x 10⁴, less than 1 x 10³, or less than 1 x 10² particles > 2 µm /ml.

The invention will now be further understood with reference to the following non-limiting examples.

### EXAMPLES

### Example 1: Effect of nebulization on aggregate formation - comparison of Fab with IgG and VHH

One of the problems of nebulizing biologicals like antibodies is the formation of aggregates. These aggregates can have different functional characteristics and cause anti-drug antibodies. Accordingly, Example 1 assesses the effect of nebulization on aggregate formation.

The aim of the present study was to evaluate the stability and biological activity of five different full-length antibodies or antibody fragments formulated in PBS containing 0.02% Tween 80, at two different concentrations after nebulization with two mesh nebulizers (commercially available).

The experiment compared the number of aggregates following nebulization of three IgG's, one Fab and one VHH. Each sample was tested at a concentration of 3 mg/ml and 10 mg/ml. Included in the experiment was the 7B07 clone in Fab format, the 7B07 clone in the corresponding IgG format, two further IgGs (10A06 clone and 08H11 clone) and a VHH (1D06 clone).

After arrival, frozen samples were kept at -20°C. Before nebulization, aliquots (of the same antibody) were pooled in a 15 mL polypropylene conical tube and the solution was stored at 4°C. Protein concentration was determined and adjusted if necessary to 3 or 10 mg/mL with PBS 0.02% Tween 80. Sample was filtered using a 0.22 µm Minisart^{®} High Flow syringe filter (Sartorius). Protein concentration, visual inspection, dynamic light scattering (DLS), flow microscopy (FCM) and size-exclusion chromatography (SEC) were performed on the sample before and after nebulization.

Nebulization was carried out using two different marketed vibrating mesh nebulizers in a basic formulation; the Aerogen^{®} Solo nebulizer and the PARI eFlow^{®} electronic nebulizer. Both devices are active vibrating mesh nebulizers that convert drug solutions into small aerosol droplets. Like other active devices, the aperture plate vibrates at a high frequency, draws the solution through the apertures in the plate and generates aerosol droplets. Each sample was nebulized in triplicate with three different Pari eFlow^{®} mesh generators. Prior to antibody nebulization, each mesh nebulizer was characterized with a 0.9% NaCl solution by laser diffraction (without the inhalation chamber and the closed-cell) and found to be comparable (volume mean diameter = 5.6 µm). Similarly, three different Aerogen^{®} Solo aerosol generator devices were used for triplicates. Before nebulizing the formulations, the three mesh nebulizers were characterized with a 0.9% NaCl solution by laser diffraction (without the closed-cell) and found to be comparable (volume mean diameter = 4.8 µm for devices s0087 and s0094, volume mean diameter = 4.9 µm for device s0210).

Each sample was nebulized and the time of nebulization was monitored. Aerosol droplets were collected using a 15 mL polypropylene conical tube (ref: 352097, Dominique Dutscher) placed directly in front of the vibrating mesh, leaving enough room for aerosol formation and allowing condensation of the nebulized solution. This additional step of condensation onto the surface of the tube may represent a stress for the protein and thus is a worst case compared to what might happen *in vivo.* The aerosol collection took place under a laminar flow.

The washing and nebulization process was similar for the 2 devices.

Steps:
1. Immerse the mesh and the other parts of the nebulizer in a bath of Surfanios premium at 5 mL/L in hot tap water, briefly mix and wait 5-10 min.
2. Rinse thoroughly the mesh and the other parts of the nebulizer with hot tap water
3. Nebulize 3 mL of hot tap water
4. Nebulize 1.2 mL (for Aerogen^{®} Solo) or 2 x 1.2 mL (for Pari eFlow^{®}) of PBS pH 7.2 filtered on 0.22 µm
5. Nebulize 1.2 mL of demineralized water filtered on 0.22 µm and collect the aerosol
6. Check by FCM that the number of particles is under the limit defined by the laboratory (<200 particles for Aerogen^{®} Solo and <300 particles for Pari eFlow^{®}). If necessary repeat from step 4 or step 1.
7. Nebulize 1.2 mL of buffer PBS 0.02% Tween 80 filtered at 0.22 µm.
8. Nebulize 1.2 ml of sample
9. Start from step 1 for another sample.

All nebulizations were achieved with the same controller: USB Aerogen or eBase Controller. These controllers dispense a continuous nebulization. The time of nebulization was measured to determine the flow rate of each device/sample (Flow rate = time of nebulization/1.2mL).

### 1.1 Granulometric Distribution

Laser diffraction experiments were performed using a SpraytecTM instrument (Malvern Instruments Ltd.). This technique was used for determining the size of the aerosol droplets generated by nebulizers. Laser diffraction permit measure of VMD (volume mean diameter), the median size of a spherical aerosol particle. 1 mL of NaCl 0.9% for device characterization or 0.5 mL of antibody solutions were used. Experiments were done at room temperature (20-25°C) and with or without a closed-cell respectively for sample or qualification. In the closed-cell, the aerosol droplets were aspirated by a constant aspiration pump at an airflow rate of 30 L/minute on the opposite side of the aerosol beam.

Aerosols were analyzed by laser diffraction. The qualification of the device with NaCl 0.9% was made without closed-cell and results were given a VMD at 4.8 and 5.6 respectively with the Aerogen^{®} Solo and the Pari eFlow^{®}.

For Ab formulation, laser diffraction was run with closed-cell, which had no impact on the VMD after nebulization with the Aerogen^{®} Solo device and reduced the VMD to 4.3 µm with Pari eFlow^{®} **(****Figure 1****).**

No significant differences of aerosol droplets size were observed between the different Ab formulations and the saline solutions (with the closed-cell).

### 1.2 Protein Concentration

Protein concentration was determined before and after nebulization by measuring absorption at 280 nm on 2 µl of solution with a Nanodrop 2000 from Thermo Fisher Scientific.

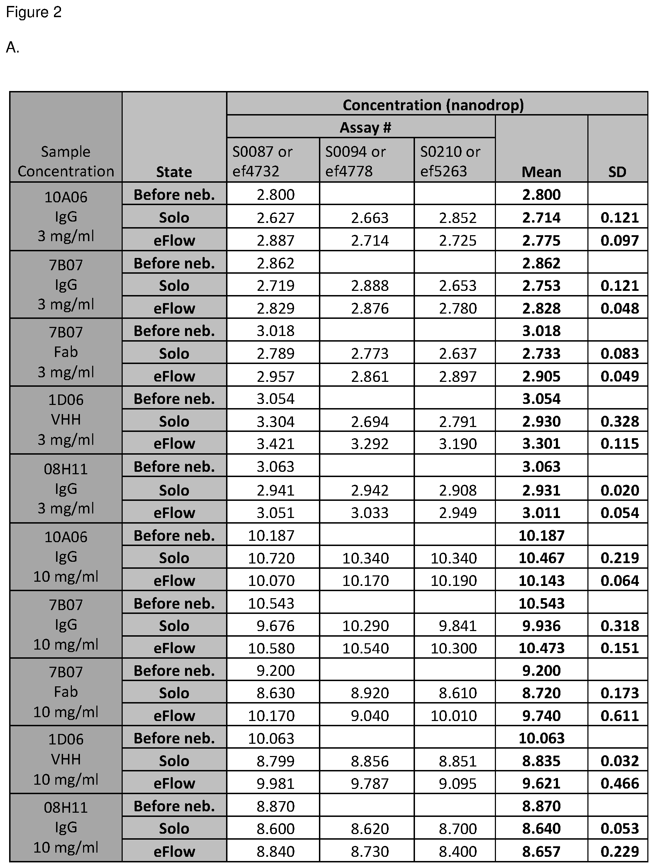

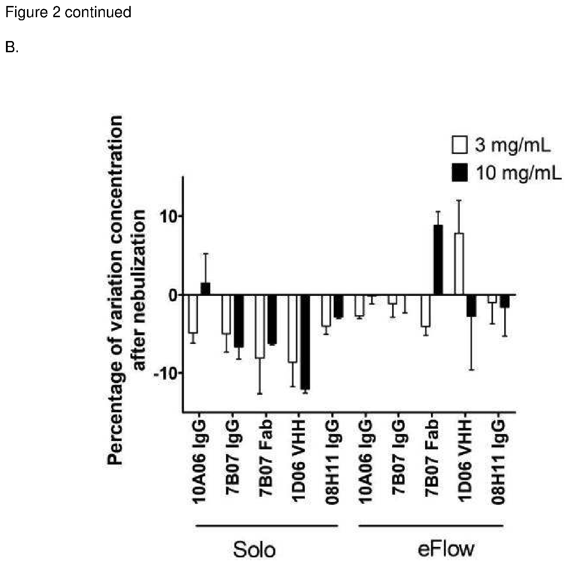

Samples were filtered prior to nebulization and protein concentration measured using a spectrophotometer. Concentrations were adjusted to 3 or 10 mg/mL, if required, before nebulization. After nebulization, protein concentrations were measured to determine any loss of biological material during nebulization. The raw data and the concentration variations before and after nebulization are shown in **Figure 2****.** Concentration varied before and after the nebulization from -12% to +12%.

### 1.3 Visual Inspection of Particles

Antibody solutions, before and after nebulization, were observed in 15 mL polypropylene conical tube for visible particles under natural light.

Before nebulization and after filtration, no particles were observed in the vials. After nebulization, visible particles were observed in most samples **(Table 2).** Notably, no particles were visible for the 7B07 Fab (contrary to the full length 7B07 IgG) after nebulization.

It is noteworthy that nebulization of the 1D06 VHH led to the formation of numerous visible particles and the solutions were cloudier compared to other samples. In some samples and with the buffer alone, foam was observed in the reservoir of the devices.

**Table 2: Visual inspection of the nebulized antibody solution**

| Sample Concentration Date | Mesh device | Visual aspect | Sample Concentration Date | Mesh device | Visual aspect |
|---|---|---|---|---|---|
| 10A06 IgG 3 mg/ml 23/05/2019 | Solo | White filaments | 10A06 IgG 10 mg/ml 29/05/2019 | Solo | White filaments |
| | eFlow | No particle | | eFlow | White particles |
| 7B07 IgG 3 mg/ml 04/06/2019 | Solo | White filaments | 7B07 IgG 10 mg/ml 11/06/2019 | Solo | White filaments or White particles |
| | eFlow | White particles | | eFlow | White filaments or White particles |
| 7B07 Fab 3 mg/ml 06/06/2019 | Solo | No particle | 7B07 Fab 10 mg/ml 12/06/2019 | Solo | 2 White filaments or No particle |
| | eFlow | No particle | | eFlow | No particle |
| 1D06 VHH 3 mg/ml 19/06/2019 | Solo | White particles, cloudy solution | 1D06 VHH 10 mg/ml 20/06/2019 | Solo | White particles, cloudy solution |
| | eFlow | White particles, cloudy solution | | eFlow | White particles, cloudy solution |
| 08H11 IgG 3 mg/ml 26/06/2019 | Solo | No particle | 08H11 IgG 10 mg/ml 27/06/2019 | Solo | No particle |
| | eFlow | No particle | | eFlow | No particle |

### 1.4 Subvisible Particles by Flow Cell Microscopy (FCM)

Flow Cell Microscopy (Flow-Imaging FC200S instrument, Occhio) is an imaging technology applied to detect and measure sub-visible and visible particles in protein-containing solutions. Before each analysis, the apparatus was washed with demineralized filtered water. To proceed further, the number of particles measured had to be <100. Before and after nebulization, 250 µL of each sample (buffer alone and antibody solution) was analysed by FCM. Mean of the number of particles with the buffer on a mesh was subtracted to the sample value. The number of particles per mL is expressed for particles with an equivalent diameter > 2 µm, >10 µm and > 25 µm.

Samples were analysed by FCM before and directly after nebulization. Results are presented in **Figure 3****.** The total number of particles should be as low as possible. Preferably, the total number of particles does not exceed 1 x10⁵ total particles/ml. The data provide evidence for the first time that biologicals in the Fab format have reduced sensitivity to aggregation during nebulization.

Before analysis, the background of the +/- nebulized buffer (PBS 0.02% Tween 80) was evaluated. Results showed before nebulization a mean of 1208 total particles. After nebulization, the mean of total particles increased to 3918 and 10203 for the Aerogen^{®} Solo device and Pari eFlow^{®} device, respectively.

Before nebulization, samples had few particles. After nebulization, the number of particles (>2µm) usually increased and depended on the samples. Overall, there were more particles at 10 mg/mL than 3 mg/mL, except for the 1D06 VHH.

For full-length IgG: The 10A06 showed the most particles (mean of 104381 and 97476 at 3 mg/mL for the Aerogen^{®} Solo and the Pari eFlow^{®} device, respectively). The 8H11 IgG produced the least particles (mean of 1,160 and 1,737 particles at 3 mg/ml and 9.744 and 776 at 10 mg/mL after nebulization for the Aerogen^{®} Solo and Pari eFlow^{®} devices, respectively). In comparison with the 7B07 IgG, the Fab format produced less particles (>2µm) after nebulization (at 3 mg/mL, 2349 and 3183 and at 10 mg/mL, 6040 and 20353 for the Aerogen^{®} Solo and Pari eFlow^{®} devices, respectively). The worst sample was 1D06 VHH which led to the most particles (>2µm) (2,149,534 and 1,874,236 particles at 10 mg/mL for the Aerogen^{®} Solo and Pari eFlow^{®} device respectively).

In most case, the level of particles was higher after nebulization with the Pari eFlow^{®} mesh compared to the Aerogen^{®} Solo device.

### 1.5 Submicronic Particles by Dynamic Light Scattering (DLS)

Dynamic light scattering experiments were performed at 25°C, with a Dynapro Nanostar^{®} instrument using a 659 nm/100 mW laser and a 90° detection angle (Wyatt Technology, Europe GmbH) to determine the size distribution profile of the solutions before and after nebulization. 100 µL of the sample was placed in a disposable single cuvette (UVette 80, Eppendorf). For each sample, we carried out 10 acquisitions of 7 seconds each. The results were analyzed with Dynamics^{®} (7.1.5.6) software. Samples with fewer than 70% of successful acquisitions were considered "not evaluable", as recommended by the manufacturer. In this case, the solutions were filtered on 0.45 µm. The dynamic data filter was set as follows: baseline limit ± 0.01 and maximum SOS 100. Results are expressed as the percentage, in mass, of the monomer and other species. Analysis gave several parameters including the hydrodynamic radius of the molecule, the polysispersity, and the polydispersity index. The polydispersity refers to the level of homogeneity of the sizes of the particles. The level of homogeneity is considered high when the percent polydispersity is less than 15%. The polydispersity index is based on a cumulants analysis. This is comparable to the distribution width divided by the mean.

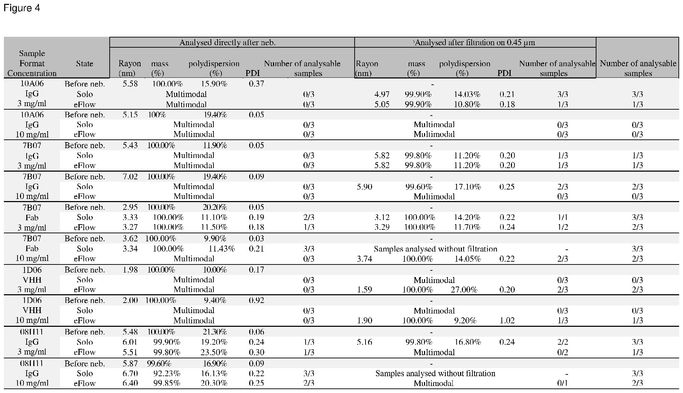

Samples were analyzed by DLS before and directly after nebulization. Results are presented in **Figure 4****.** Before nebulization, the percentage mass of the main species in the solution (main peak) were comparable for all formulations. The radius of the main peak was in agreement with the size of the protein.

After nebulization, most samples could not be analysed by DLS, probably due to the amount of large particles (as demonstrated by FCM). The samples that could be analysed were 08H11 IgG at 3 and 10 mg/ml, 7B07 Fab at 3 mg/ml for the two devices, and 7B07 Fab at 10 mg/ml after nebulization with the Aerogen^{®} Solo device.

Samples were analysed after filtration on 0.45 µm when they gave a multimodal result without filtration. However, applying filtration did not improve the DLS results, since a large part of the samples remained multimodal after filtration. No significant variations of the percentage of polydispersion and the PDI were observed. Overall, the DLS results were in agreement with the FCM ones.

### 1.6 Size Purity by SEC-HPLC

After sample dilution to 1 mg/ml and filtration on 0.22 µm, size-exclusion chromatography (SEC) analyses were performed on an UltimateTM 3000 UHPLC system equipped with an in-line. For each sample, 50 µg was injected by an UltimateTM autosampler in an Agilent Technologies (Santa Clara, CA) Bio SEC-3 column (3 µm, 300 Å, 7.8 x 300mm), and separated at a flow rate of 1 mL/min. The elution buffer was PBS pH 7.2 and UV detection was performed at 280 nm with an UltimateTM diode array detector (Thermo Fisher Scientific). Results are expressed as the percentages of monomers and higher molecular weight species (HMWS).

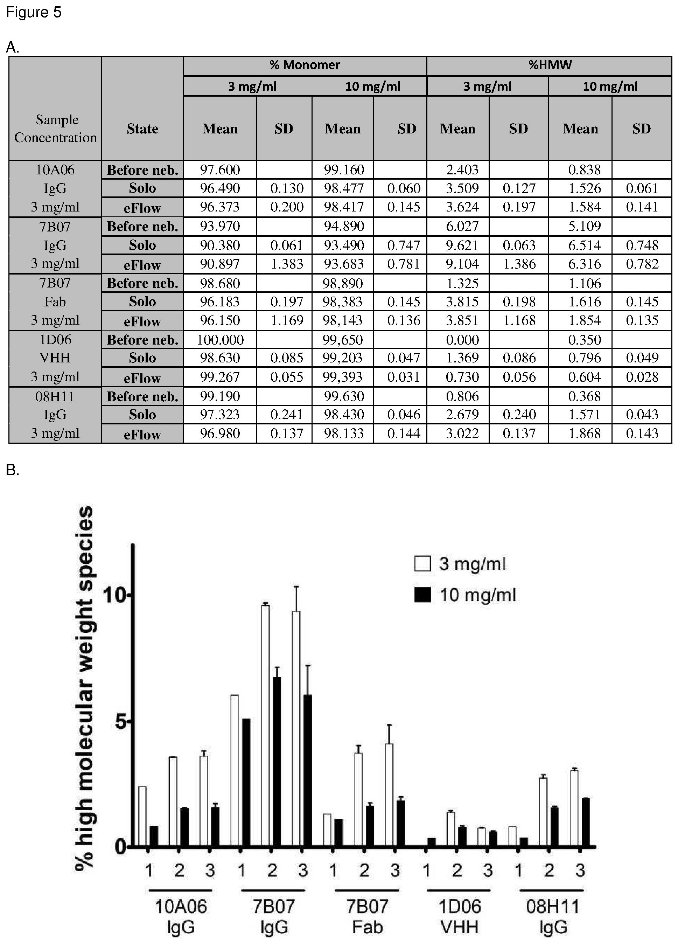

Samples were analyzed by size exclusion chromatography before and after nebulization. Results are presented in **Figure 5****.** Before nebulization, the percentage of mass of the main species in the solution (main peak) were variable for all formulations and concentrations. The 7B07 IgG showed a monomer peak inferior to 95%. After nebulization, the percentage of monomers decreased in some samples. For full-length IgG, the nebulization of 7B07 showed the most significant loss, in particular at 3 mg/mL. In most cases, the device had no impact.

### 1.7 Summary

The aim of this study was to assess the stability of different anti-galectin-10 Abs during nebulization. Three IgG, one Fab and one VHH were studied. The results showed that after nebulization the profile of aggregation is dependent on the antibody. The 8H11 IgG and the 7B07 Fab were the most stable during nebulization, inducing less aggregation. The 1D06 VHH was the most unstable, leading to high levels of aggregation.

Sample were nebulized on two mesh nebulizers: Aerogen^{®} Solo device and Pari eFlow^{®}. No significant difference of aggregation profile were observed between the two systems, although the Aerogen^{®} Solo device has a tendency to produce less aggregates. Nebulization of the sample showed the Aerogen^{®} Solo device induced a light decrease of protein concentration, had a lower flow rate and a size of droplets slightly higher than the Pari eFlow^{®}.

Two concentrations of samples were nebulized: 3 and 10 mg/ml. More subvisible aggregates (FCM) were usually observed at 10 mg/ml than at 3 mg/ml. Overall, the data showed that among the five tested molecules, the 7B07 Fab form was the most stable during nebulization. Indeed, the nebulization of this antibody fragment induced few particles after collection using methods that scanned different aggregate populations.

### Example 2: Effect of nebulization on the stability of various Fab fragments

This study evaluated several physicochemical characteristics of four anti-galectin-10 Fab clones able to solubilize CLCs. The molecules assessed in Fab format were the clones: 18C06, 20H09, 23H09 and 24F02_N53A (see Example 3 below for further details of Fab production and characterisation).

Clone 7B07_N53A in Fab format was also included as a comparator. Fab 7B07_N53A is related to Fab 7B07 tested in Example 1 above but was engineered to include a N53A mutation, to mitigate a deamidation liability. Fab 7B07_N53A was found to be less stable than Fab 7B07 but was found to be fully potent for dissolving in vitro CLCs. Therefore, 7B07_N53A was included for selected conditions and time-points for comparison purposes.

All four clones described in this study share a common VH+CH1, CH2 and CH3 part, three clones (20H09, 23H09, and 24F02_N53A) share a common light chain which is the parental LC of 7B07_N53A, while one clone (18C06) has its own light chain.

Endotoxin free germlined Fabs were produced by U-Protein Express (UPX), Utrecht, The Netherlands. The anti-galectin-10 Fab molecules were purified and delivered to argenx by UPX in Dulbecco's PBS buffer containing 0.02% Polysorbate20. The proteins and formulation buffers were delivered as 0.22 µm-filtered material. The produced material was stored at UPX at 2-8°C and shipped to argenx at 2-8°C. Upon arrival, the purified material of all clones were stored at 2-8°C for up to 48h and the protein concentration was adjusted to 10 mg/mL in the original formulation buffer under aseptic conditions.

All four Fabs were evaluated for their stability after nebulization. The nebulization stress and its impact on the physicochemical properties of the clones was evaluated before (T0) and after 4 weeks storage at +5°C (T4W) with the use of triplicate mesh nebulizers. All aerosolized solutions (including formulation solutions) were fully characterized at both time points.

Each clone was nebulized with the Aerogen Solo active vibrating mesh nebulizer that converts drug solution into an inhalable aerosol. Three different devices for each time-point were used per clone and results are reported by the device (nebulizer) serial number. In case of device fouling, indicated by longer nebulization times than usual for a given size of aliquot, spare devices of the same type were available to complete the nebulization with triplicate devices.

Clones 18C06, 20H09, 23H09 and 7B07_N53A were nebulized with the Aerogen Solo devices with serial numbers #1690, #0125 and #0259. Clone 24F02_N53A, which was nebulized in a later stage, was evaluated after nebulization with the same devices but as some fouling was observed for #0125, an additional device with number #1512 was used. To bridge results for the aerosolized proteins with this fourth device used in the study, aliquots from clones 20H09 and 23H09 were also nebulized with this fourth device along with clone 24F02_N53A. Results are illustrated as the mean of triplicate measurements per device unless stated otherwise.

A tabulated summary illustrating several core characteristics of the Fabs tested (before any type of stress applied) is provided in **Table 3.**

**Table 3: Overview of anti-galectin-10 Fab attributes**

| **Attribute** | **Criteria (Target)** | **g18C06** | **g20H09** | **g23H09** | **g24F02_N53A** | **(g7B07_N53A)** |
|---|---|---|---|---|---|---|
| **Format** | **Fab** | Fab | Fab | Fab | Fab | Fab |
| **Human identity** | **% identity to human germ V-region** | 100% | 98.8% | 98.8% | 100% | 98.8% |
| **Immunogenicity** | **Epibase score: human** | Human | Human | Human | Human | NT |
| | **Immunogenicity score** | Low | Low | Low | Low | |
| **Cross-reactivity** | **Cyno cross-reactivity** | No | No | Yes | Yes | No |
| **Binding activity** | **KD (affinity)** | Yes | No | Yes | Yes | No |
| | **≤15 nM** | (1.3 nM) | (17.7 nM) | (5.3 nM) | (1.4 nM) | (145.0 nM) |
| | **kd (off rate)** | Yes | No | Yes | Yes | No |
| | **≤5E-03/s** | (4.9E-04/s) | (4.7E-02/s) | (3.1E-03/s) | (1.8E-03/s) | (79.0E-03/s) |
| **Isoelectric point (pI)** | **Report result** | 9.55 | 8.32 | 7.47 | 8.03 | NT |

| | | | | | | |
|---|---|---|---|---|---|---|
| NT: not tested | | | | | | |

### 2.1 Protein Concentration

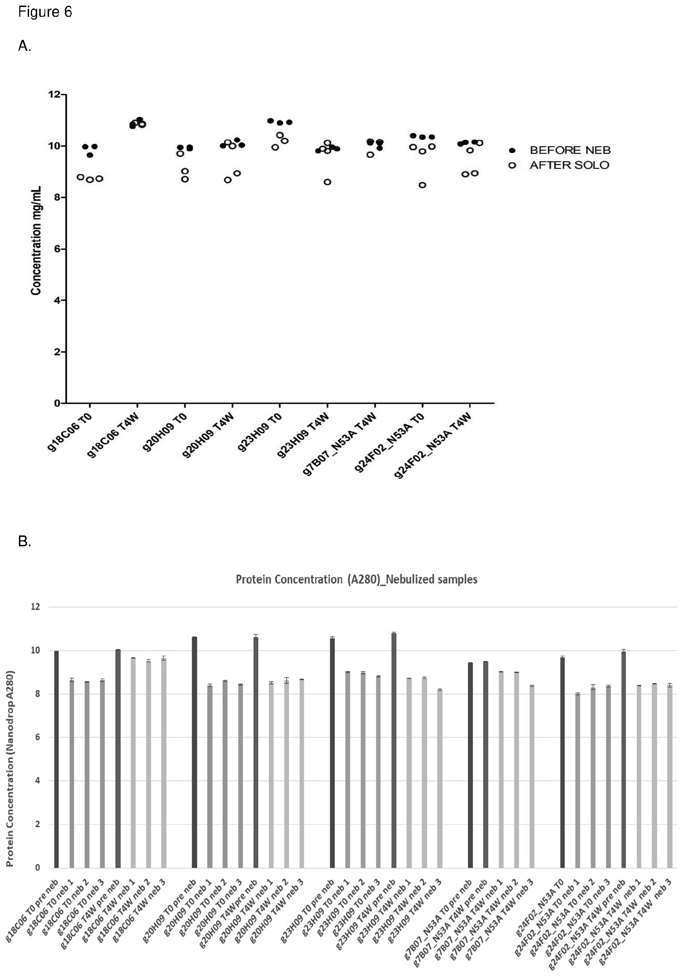

Protein concentration was evaluated at A280nm (Nanodrop). The protein concentration for all aerosolized solutions presented a clear drop for all clones. As illustrated in **Figure 6****,** this was the case for all nebulizers used for both time-points, T0 and T4W after storage under refrigerated conditions. This trend was confirmed by independent measurements.

### 2.2 Visible Inspection of Particles

A visual inspection of the samples took place by two individuals for all clones, at both time-points T0 and T4W, before and after nebulization (0.5mL fill in 1.5mL in transparent, glass vials). Before nebulization, the solutions were inspected for the presence of visible particles but no particles could be seen for any of the clones inspected. T0 aerosolized solutions presented no visible particles except for clone 23H09. For the 4W time-point, white particles were observed in the post-nebulization aliquots of 23H09 and 24F02_N53A, while 18C06 and 20H09 were found particle-free. Aerosolization induced foaming upstream of the mesh vibrating module, in the reservoir **(Table 4).**

**Table 4: Visual inspection of clones before and after mesh nebulization**

| **Sample** | **Observation prior to nebulization** | **Aerogen Solo device serial no.** | **Observation liquid post-nebulization** | **Observation device post-nebulization** |
|---|---|---|---|---|
| **g18C06 T0** | No aggregate. Clear | 1690 | No aggregate. Clear | Foam upstream the mesh, in the reservoir |
| | | 0125 | No aggregate. Clear | Foam upstream the mesh, in the reservoir |
| | | 0259 | No aggregate. Clear | Foam upstream the mesh, in the reservoir |
| **g18C06 T4W** | No aggregate. Clear | 1690 | No aggregate. Clear | Foam upstream the mesh, in the reservoir |
| | | 0125 | No aggregate. Clear | Foam upstream the mesh, in the reservoir |
| | | 0259 | No aggregate. Clear | Foam upstream the mesh, in the reservoir |
| **g20H09 T0** | No aggregate. Clear | 1690 | No aggregate. Clear | Foam upstream the mesh, in the reservoir |
| | | 0125 | No aggregate. Clear | Foam upstream the mesh, in the reservoir |
| | | 0259 | No aggregate. Clear | Foam upstream the mesh, in the reservoir |
| **g20H09 T4W** | No aggregate. Clear | 1690 | No aggregate. Clear | Foam upstream the mesh, in the reservoir |
| | | 0125 | No aggregate. Clear | Foam upstream the mesh, in the reservoir |
| | | 0259 | No aggregate. Clear | Foam upstream the mesh, in the reservoir |
| **g23H09 T0** | No aggregate Clear | 1690 | White aggregates (+) | Foam upstream the mesh, in the reservoir |
| | | 0125 | No aggregate. Clear | Foam upstream the mesh, in the reservoir |
| | | 0259 | White aggregates (+) | Foam upstream the mesh, in the reservoir |
| **g23H09 T4W** | No aggregate Clear | 1690 | No aggregate. Clear | Foam upstream the mesh, in the reservoir |
| | | 0125 | No aggregate. Clear | Foam upstream the mesh, in the reservoir |
| | | 0259 | White aggregates (+) | Foam upstream the mesh, in the reservoir |
| **g7B07_N53A T4W** | No aggregate Clear | 1690 | White aggregates (+) | Foam upstream the mesh, in the reservoir |
| | | 0125 | White aggregates (+) | Foam upstream the mesh, in the reservoir |
| | | 0259 | White aggregates (+) | Foam upstream the mesh, in the reservoir |
| **g24F02_N53A T0** | No aggregate Clear | 1690 | No aggregate, Clear | Foam upstream the mesh, in the reservoir |
| | | 0125 | White aggregates (++) | No observation |
| | | 0259 | No aggregate, Clear | Foam upstream the mesh, in the reservoir |
| | | 1512 | No aggregate, Clear | Foam upstream the mesh, in the reservoir |
| **g24F02_N53A T4W** | No aggregate Clear | 1690 | White aggregates (+) | Foam upstream the mesh, in the reservoir |
| | | 0125 | White aggregates (++) | No observation |
| | | 0259 | White aggregates (+) | Foam upstream the mesh, in the reservoir |
| | | 1512 | White aggregates (+) | Foam upstream the mesh, in the reservoir |

| | | | | |
|---|---|---|---|---|
| +: 2-5 aggregates; ++: 5-15 aggregates; Solo 0125 is the device where aberrant results have been observed. | | | | |

### 2.3 Subvisible Particles by Dynamic Light Scattering (DLS)

DLS analysis was carried out for all clones and formulation samples at both time-points T0 and T4W before and after nebulization. Measurements took place in triplicate preparations with a DynaPro Nanostart instrument. Percent mass, hydrodynamic radius of molecule, percent polydispersity (%PD) and polydispersion index (PDI) were used to monitor the distribution profiles of the submicron particles in solution. All sample solutions at both time-points were filtered (0.2 µm) before and after nebulization in order to carry out the analysis.

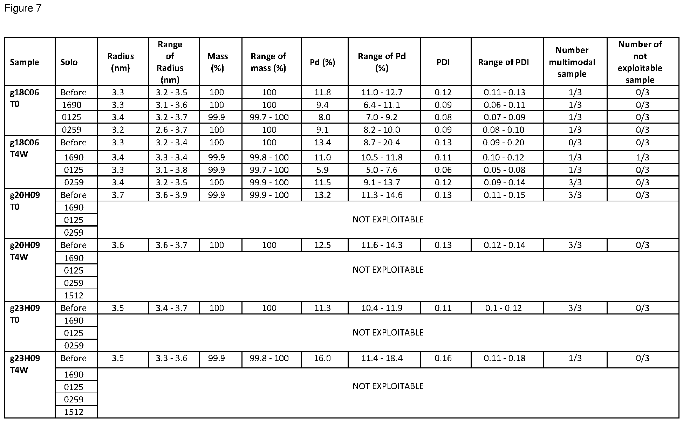

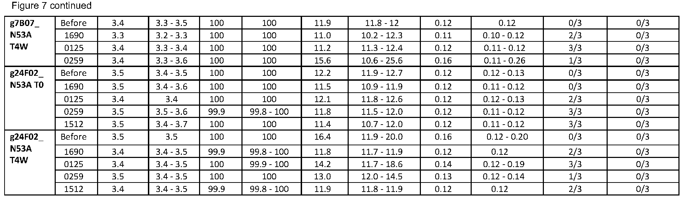

Evaluation was done by comparing the nebulized proteins before and directly after nebulization **(****Figure 7****).** Before nebulization, the percentage in mass of the main species in the solution (monomer) for all clones was over 99.9%. Similarly, for clones 18C06 and 24F02_N53A (along with 7B07_N53A), the percentage in mass of the main species was greater than 99.9% following nebulization, and the presence of non-monomeric species was negligible. The data for clones 20H09 and 23H09 post-nebulization was not exploitable due to elevated particle levels. The radius of the main peak was in agreement with the expected size of the protein for all Fabs.

### 2.4 Subvisible Particles by Flow Cell Microscopy (FCM)

Flow Cell Microscopy (FCM) was carried out with a Flow-Imaging FC200S instrument, Occhio in order to detect and measure 1 to 100 µm particles in solutions. Before each analysis, the apparatus was extensively washed with demineralized filtered water aliquots. As a system suitability test, each analysis took place once the number of particles in these aliquots was measured to be < 100 total counts. The number of particles per mL is expressed as total particles and as particles with an equivalent diameter > 2 µm, >10 µm and > 25 µm.

All clones were measured prior and after nebulization for time-points TO and after 4-weeks storage at 5°C (T4W). As above, all samples were filtered (0.2µm) in order to complete the assessment. Buffer samples for both time-points were filtered as well (before and after nebulization). All FCM values herein represent the average value obtained from the three devices used (triplicate measurements per device) after subtracting the average value of particles (total and per category) of the formulation buffer from all devices (triplicate measurements per device).

Fab clones 18C06, 20H09, 23H09 and 7B07_N53A were nebulized all together in a first wave, while Fab clone 24F02_N53A was nebulized in a later stage after replacing the fouled device (#0125). Solutions from the first wave of clones, wherever they were available, were also nebulized with the new device used for clone 24F02_N53A (#01215) in order to bridge the nebulization waves and results.

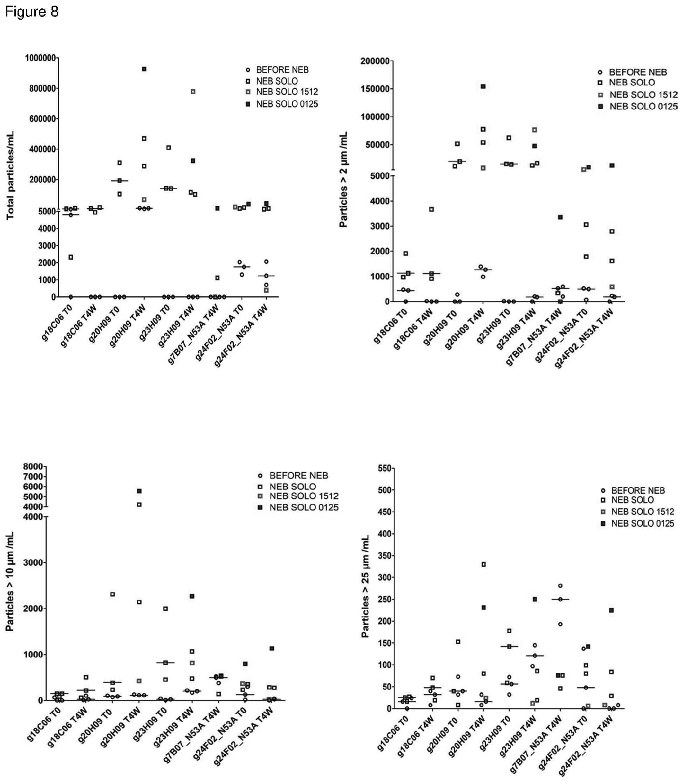

As illustrated in **Figure 8****,** Fabs 18C06 and 24F02_N53A, at both T0 and T4W (along with 7B07_N53A), presented fewer particle counts in all size categories after nebulization as compared to the other samples. The subvisible counts obtained from the nebulization experiments are illustrated in **Table 5** and focus on the total counts/mL and > 2µm particles/mL, as this last category comprises the particles which can potentially induce immunogenicity upon drug administration of the inhaled medication.

Subvisible and submicron particles testing demonstrated the need for formulation optimization. Clone 24F02_N53A presented with relatively lower and more stable particle counts for all categories after storage for 4 weeks and nebulization. Clone 23H09 demonstrated relatively higher counts in all categories after nebulization when compared to clone 24F02_N53A, including the particles with size >2µm, a category which is often associated with immunogenicity and severe ADA responses which might jeopardize the drug safety, efficacy and pharmacokinetics.

**Table 5: Overview of subvisible particles results expressed as total particles/mL and >2µm particles/mL**

| * the asterisk indicates the cumulative counts including device #0125 which presented fouling leading to aber results rant | | | | | |
|---|---|---|---|---|---|
| **Timepoint** | **g18C06** | **g20H09** | **g23H09** | **g24F02_N53A** | **g7B07_N53A** |
| **Total Particles/ mL** | | | | | |
| **T0** | 6,547 | 1,476 | 302 | 2,456 | NA |
| **T0 post-neb.** | 16,201 | 212,723 | 241,257 | 26,741 */ 21,550 | NA |
| **T4W at +5°C** | 266 | 14,063 | 854 | 2,606 | 1,827 |
| **T4W post-neb.** | 23,363 | 452,413*/ 291,462 | 302,794 */ 292,352 | 25,941 */ 19,713 | 12,104 |

| **>2µm particles/ mL** | | | | | |
|---|---|---|---|---|---|
| **T0** | 559 | 292 | 139 | 642 | NA |
| **T0 post-neb.** | 2,204 | 28,532 | 31,724 | 6,160* / 4,721 | NA |
| **T4W at +5°C** | 137 | 1,576 | 397 | 442 | 802 |
| **T4W post-neb.** | 3,992 | 75,862 */ 49,344 | 37,270 * /32,911 | 7,111 * /4,328 | 3,118 |

### 2.5 Galectin-10 Binding Activity by Surface Plasmon Resonance (SPR)

The functional active concentration of the nebulized solutions was assessed by SPR on a Biacore 3000 instrument. All samples were evaluated with methods meeting the standard qualification criteria. Aliquots were tested at predefined test concentrations against titration curves from reference samples (T0) and in the presence of quality control (QC) samples.

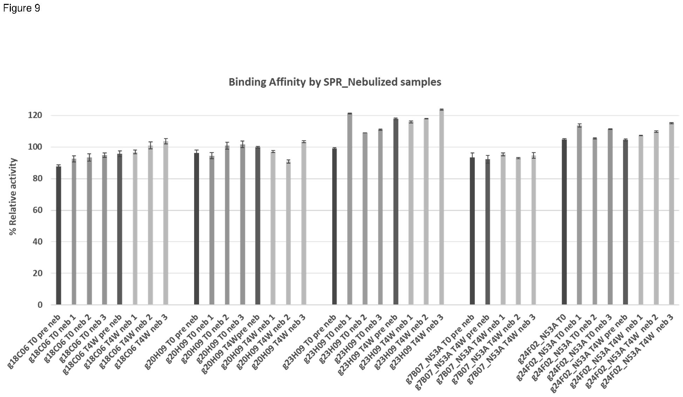

As illustrated in **Figure 9****,** all nebulized clones were found to be fully functional without any loss in percent relative activity towards the unstressed reference material of each clone. The nebulization stress had no impact on the activity of the clones neither at TO nor after 4 weeks storage at +5°C.

### 2.6 Size Purity by SE-HPLC

Purity by SE-HPLC was assessed with an Agilent 1260 Infinity II chromatographic system, equipped with a quaternary pump, automatic injector, on-line degasser and a DAD detector, column thermo-stated compartment (21°C), and autosampler set at 6°C. The detector was set to wavelengths 280 nm and 214 nm simultaneously to monitor size variants. Samples were analyzed before and after nebulization for both time-points, TO and T4W.

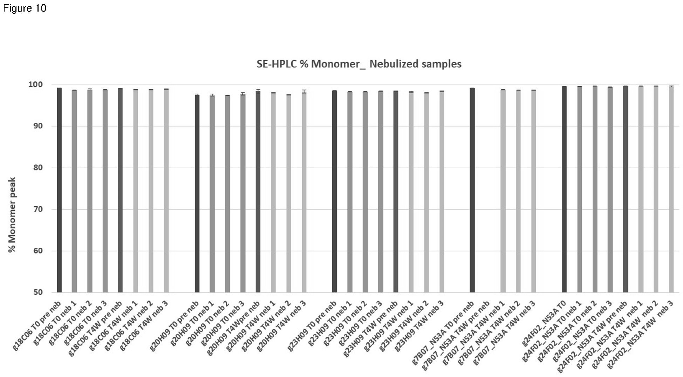

As illustrated in **Figure 10****,** all four Fab clones were measured with more than 97% purity after nebulization regardless of the time-point or device used. The percentage total aggregation (%HMW species) and total fragmentation (%LMW species) remained at very low levels in all cases i.e. below 3% as total non-monomeric percentage.

All aliquots were filtered prior to nebulization for both time-points T0 and T4W. This was carried out as the solutions presented elevated counts of particles via DLS measurements (Section 2.3). As a consequence, the SEC results illustrated herein for the nebulized clones should be interpreted mainly in a qualitative approach. The figures demonstrate relative stability for all clones after mesh nebulization even after 4 weeks storage under refrigerated conditions.

### 2.7 Purity by cGE

Purity by cGE was assessed upon lab-on-a-chip analysis using an Expert 2100 Bioanalyzer device (Agilent). Samples were analyzed under reducing and non-reducing conditions before and after nebulization.

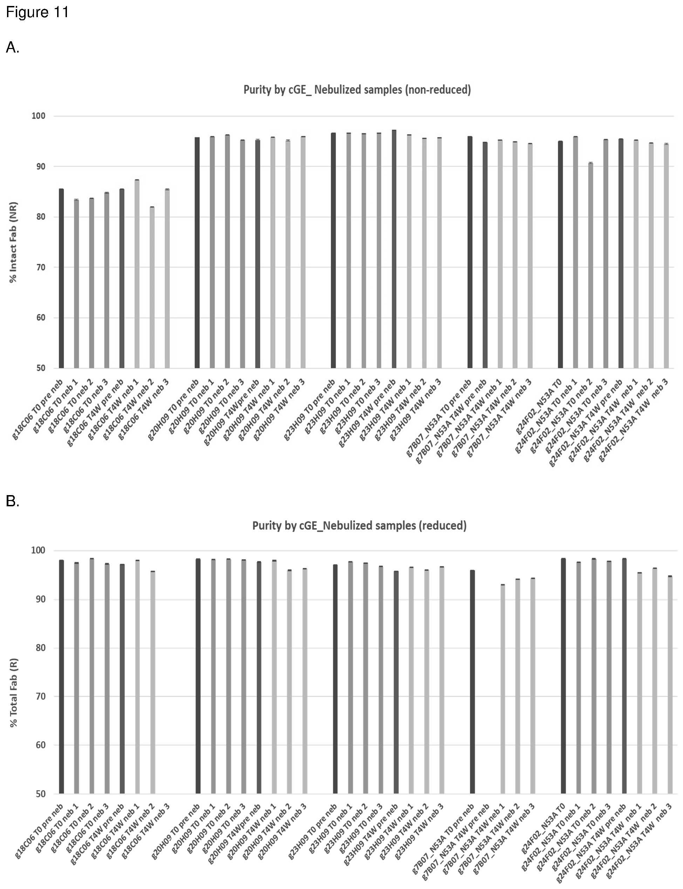

As illustrated in **Figure 11****,** the applied nebulization stress had no impact on the Fab clone purity, except for clone 18C06, which had the lowest purity readout. This is in accordance with all previous analyses, even for the non-stressed material. This clone failed to demonstrate purity of the main peak of more than 90% under non-reducing conditions but there was no further impact by nebulization. For the rest of the clones, purity exceeded 90% under non reducing conditions regardless the nebulization time-point **(****Figure 11****,** upper panel). Under reducing conditions, all molecules demonstrated more than 95% purity **(****Figure 11****,** lower panel).

### 2.8 Post-translational Modifications (PTMs)

The structural characterization of all Fab clones was performed at protein and peptide level using several analytical techniques (icIEF, online-desalting MS, RPLC-UV-MS on reduced protein, peptide map using RPLC-MS after tryptic digestion). The clones were analyzed for PTMs after nebulization at both T0 and after 4W storage at +5°C (T4W). In all cases, the analyses were carried out side-by-side with the control unstressed reference material (pre-nebulization) for each clone.

No major issues were picked up for the clones after nebulization. The main findings are summarized below:
▪ The amino acid sequence of the four Fab clones was confirmed at the protein level, based on the molecular weight of each intact Fab (LC and VH+CH1) while the peptide sequence coverage was 100%.
▪ The structural integrity before and after stress of the Fabs remained unchanged and was confirmed with intact protein and peptide mapping analyses which demonstrated and confirmed the presence of the (inter- and intra-chain) expected disulfide bridges. Only 18C06 (all samples including reference) was detected with a free cysteine in the LC instead of the expected bridge with the VH+CH1 part and instead, an alternative formation of a disulfide bridge between two closely located cysteines in the LC was detected.
▪ Oxidation after nebulization remained < 1% for all clones.
▪ Site specific events such as isomerization and deamidation remained generally unaffected after nebulization for all clones.

### 2.9 Potency - Crystal Dissolution Assay

During this study, all Fab clones were evaluated for their capacity to dissolve GAL10 crystals in vitro. For this reason, a crystal dissolution assay (CDA) was developed and standardized at Charles Rivers Laboratories (CRL), Leiden, The Netherlands, for assessing the biological activity of the Fab clones prior to and after nebulization. The objective was to evaluate if the nebulization prior/after storage could have an impact on the (bio)activity of the clones leading to a loss in potency for the dissolution of GAL10 crystals.

For all Fabs, the analysis was performed in two independent experiments with the presence of the appropriate assay controls. For the nebulized clones with triplicate devices, the aerosolized protein from one common device was analysed as 8-concentration points curve (Solo #0125) and the remaining two at a preselected, fixed concentration, always in two independent assays. Samples after 4 weeks storage at +5°C were analysed as 8-concentration points curve for all clones. Results illustrated herein are taken from assays for which the size distribution of the crystals was more than 10µm (10-15 µm).

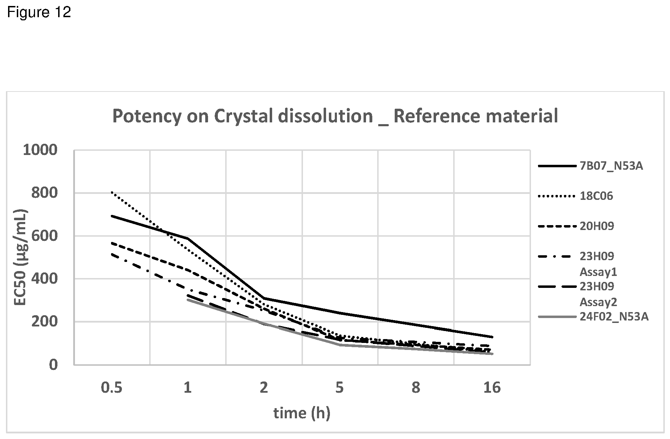

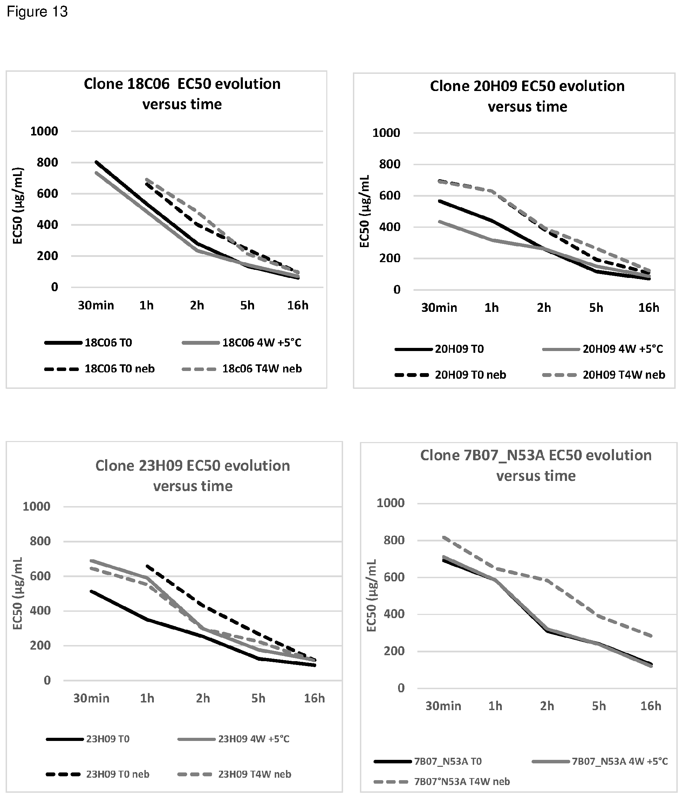

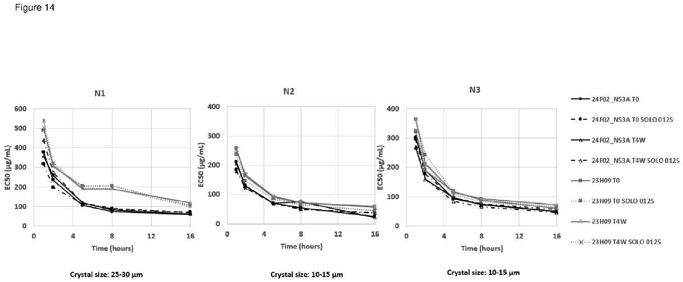

**Figure 12** illustrates the potency of unstressed samples of the four Fab clones to dissolve GAL10 crystals. Alongside the non-stressed material, all clones were tested for potency after undergoing the stress conditions described above to evaluate if they could preserve this potency after storage and/or after nebulization. Clones 18C06, 20H09 and 23H09 were tested in parallel on a first wave (assay 1, **Figure 13****),** and 7B07_N53A clone was used for comparison. Clone 24F02_N53A was analysed side-by-side with clone 23H09 on a second wave (assay 2, **Figure 14****).**

As illustrated in **Figure 12****,** **Figure 13** **and** **Figure 14****,** all clones, regardless of the stress applied, have the potency to dissolve fully the Galectin-10 crystals throughout the analytical run. Consequently, the comparison of the clones focused mainly on the first time-points up to 5h where a differential in potency could be picked up and evaluated. Clones 23H09 and 24F02_N53A were the most potent molecules amongst the four clones in the assays conducted. **Figure 14** illustrates the results obtained from assay 2 with side-by-side comparison of these two most potent clones. For all independent runs and regardless of the size distribution of the crystals plated, clone 24F02_N53A seemed always more potent for dissolving the crystals in vitro. The temperature or nebulization stress did not affect its capacity for dissolution when compared to the unstressed material. Based on this assay monitoring the potency for crystal dissolution, the clones can be ranked from the most to least potent as:
▪ 24F02_N53 >23H09 > 20C06> 18C06

Clone 24F02_N53A showed some benefit over 23H09; though both clones were able to eliminate the crystals in vitro, when these two clones were tested side-by-side on two independent occasions, 24F02_N53A presented a faster dissolution rate and elimination of the crystalized protein.

### 2.10 Immunogenicity

Endotoxin-free material for all clones was sent to Lonza, Slough, UK in order to assess the immunogenicity risk using Lonza Epibase^{®}, which includes an *in silico* and a cell based *in vitro* assessment.

The *in silico* assessment of the Lonza Epibase^{®} uses an algorithm to screen the amino acid sequences of the clones for potential immunogenic epitopes, including the allotypes that can be affected and the major histocompatibility complex by measuring the HLA-DRB1 score.

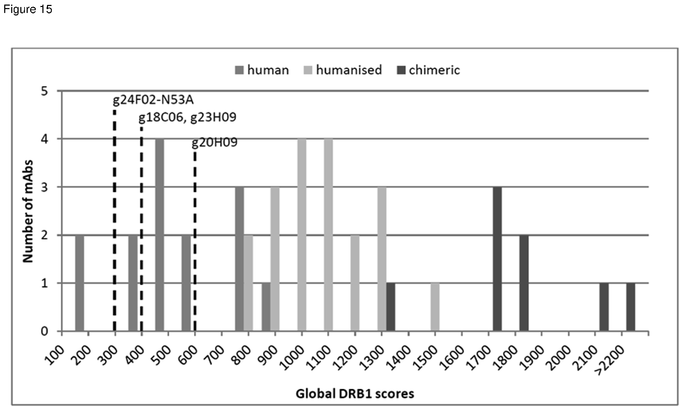

The assay screened these allotypes next to their global population frequencies **(****Figure 15****)** and based on their immunogenicity score, the clones were ranked from the least to the most immunogenic as:
▪ g24F02_N53 < [g23H09, g18C06] < g20H09

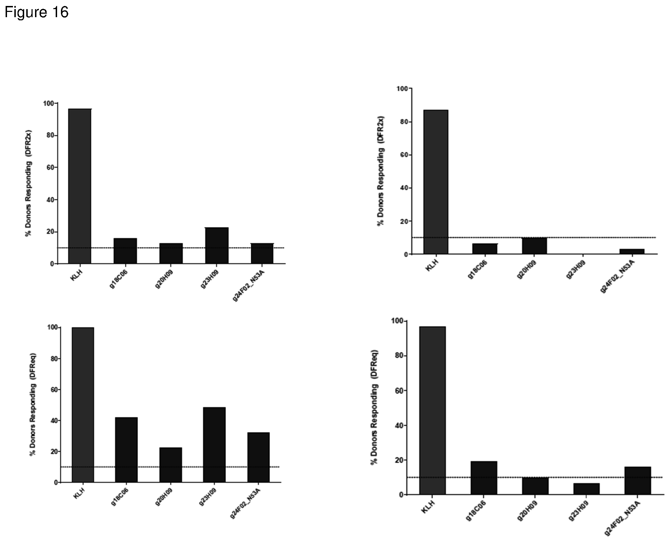

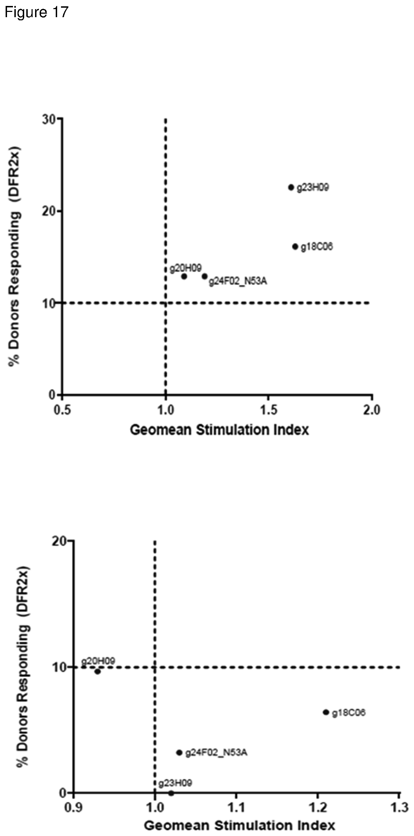

These results were also confirmed with the Lonza Epibase *in vitro* assay. In short, the clones were evaluated for T cell responses induced in PBMCs from 31 healthy donors. Screening was evaluated upon detection and enumeration of stimulated IFNγ and IL-5 cells to determine the number of donors eliciting a T-cell response as unwanted immune response risk **(****Figure 16****),** and the magnitude over the test population **(****Figure 17****).**

As shown in the corresponding Figures **(****Figure 16****,** **Figure 17****),** all Fabs demonstrated a low capacity to induce immune responses. Amongst the four, in terms of IFNy response, clones 23H09 and 18C06 demonstrated the highest frequency, while in terms of IL-5 responses, Fabs 18C06 and 20H09 demonstrated the highest frequency. In all cases and with all statistical approaches, clone g24F02_N53A was the molecule with the least frequency to induce an unwanted T-cell response and thus, this clones is considered to be the clone with the lowest risk.

### 2.11 Summary of Results

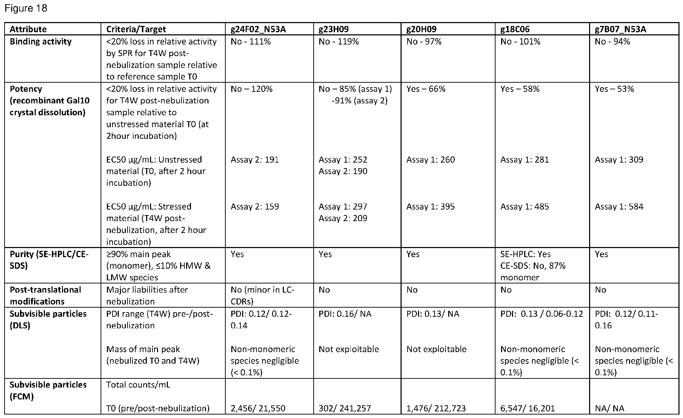

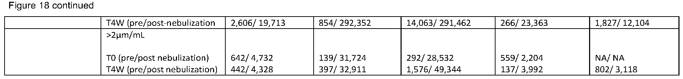

Four germlined Fab clones were evaluated, and all four clones demonstrated improved characteristics compared to the 7B07_N53A clone from which they were derived. A summary of the results is provided in **Figure 18****.**

Nebulization of all clones did not significantly impact protein concentration, purity by SE-HPLC and cGE (reduced and non-reduced), binding activity by SPR, post-translational modifications and in vitro galactin-10 crystal dissolution. Based on the stability data obtained for nebulization before and after a storage period of 4 weeks, all clones demonstrated comparable attributes without any major deviations from the target criteria set.

Concerning the immunogenicity of the anti-GAL10 Fab clones, the related risk assessment showed an advantage for clone 24F02_N53A, which is also more favourable in terms of lower immunogenicity score, although the difference with 23H09 is limited. The same picture is obtained when the two clones are compared for their human identity **(Table 3)** with clone 24F02_N53A presenting 100%.

Subvisible and submicron particles analyses (by DLS and FCM) demonstrated the need for formulation optimization **(****Figure 7****)** as even unstressed samples for clone 23H09 (and also 20H09) were found highly heterogeneous, multimodal and non-exploitable. The nebulization amplified these phenomena for all particles categories, with clone 24F02_N53A presenting relatively low and stable numbers after storage for 4 weeks and nebulization. Clone 23H09 demonstrated higher counts in all categories after nebulization when compared to 24F02_N53A **(Table 5)**. Though there is a need to optimize the formulation to be aerosolized for drug delivery through inhalation, overall clone 24F02_N53A was found more favourable with lower particle counts, especially for the category of particles with size >2µm, a category which is often associated with immunogenicity and severe ADA responses which might jeopardize the drug safety, efficacy and pharmacokinetics.

From the potency results obtained before any nebulization stress **(****Figure 12****)** and after storage and aerosolization with a vibrating mesh nebulizer **(****Figure 14****),** clone 24F02_N53A had an advantage over 23H09. For all samples tested side by side regardless of the assay, 24F02_N53A demonstrated faster potency on dissolving galectin-10 crystals than 23H09, although all clones were able to eliminate the crystalized protein in vitro.

### Example 3: Generation and characterization of the anti-galectin-10 Fab fragments

In a previous discovery campaign, the clone 7B07 was identified. This clone showed the best potency to dissolve recombinant CLCs and demonstrated appropriate binding properties. However, further stability studies highlighted a deamidation site (N53G54) in the CDR2 of the heavy chain responsible for a dramatic drop in binding and potency after incubation at 37°C. Variants of the germlined 7B07 (g7B07) clone were generated in which N53 and G54 were randomized, but all mutations, including the 7B07_N53A clone, resulted in a clear drop in binding properties. However, the potency of these mutant 7B07 clones to dissolve recombinant CLC was preserved.

Consequently, three discovery campaigns were initiated to identify an anti-Gal10 clone with good potency, affinity, and stability. The campaigns aimed to find variant clones with binding to a similar epitope as 7B07, as this clone appeared to be the most potent clone and binds to a unique epitope. The assumption was that if a clone binds to a similar epitope as 7B07, similar potency will be achieved. The first campaign aimed to find a clone in the immune libraries available that binds to a similar epitope ("7B07 epitope campaign"). In the second campaign, sequences of the CDR2_VH were randomized at up to 6 positions, including N53 and G54, to identify clones with good binding characteristics and without the deamidation site ("g7B07_CDR2_VH randomization campaigns"). In a third campaign, the heavy chain shuffling approach was executed to find clones that pair with the 7B07 light chain and allow for good affinity to Gal10 and improved stability ("Heavy chain shuffling campaign").

Following the discovery campaigns, four clones were identified for further study: 18C06, 24F02_N53A, 23H09 and 20H09.

### 3.1 Germlining, reformatting and production of the clones in the human Fab backbone

The four clones were selected to be humanized and recloned in human Fab backbone for further characterization. To reduce the immunogenicity of the anti-Gal10 clones derived from llamas, the germlining of the variable regions (VH and VL) was initiated through complementarity determining region (CDR) grafting into the closest human germline framework (FW). AbAligner and Antibody extractor software version N° 8.1 were used to identify the human germline sequence with the highest identity of the V region of the selected clones. The variant 24F02 was engineered to remove a potential deamidation site (pos53_CDR2_VH) located at exactly the same position as 7B07. For this reason, the N position 53 was mutated to A.

### 3.2 Confirming binding properties of the Fab clones

The binding properties of the selected Fab clones to human Gal10 were analyzed via a capture method established on Biacore 3000. It was decided to apply two concentrations of the selected Fab clones on human Gal10-His immobilized on a CM5 chip coated with a monoclonal anti-His. As controls, the clone 7B07 and 7B07_N53A were injected at the beginning and the end of the run.

The selected clones isolated from the three different discovery campaigns showed similar or better off rate and affinity than clone 7B07. Among this panel, the clone isolated from the 7B07 epitope campaign (18C06) showed the best off rate with a 9.8 fold better off rate than clone g7B07. The unique clone isolated from the heavy chain shuffling approach (g24F02) and its engineered variant (g24F02_N53A) showed highly similar binding capacity, demonstrating that the removal of the deamidation site found at position 53 does not impact its binding properties. With an on rate between 7.1-8.3 E+05, and off rate equal to 1.6E-03 1/s, and an affinity between 2.0-2.3 nM, these clones showed 1.6 to 1.9 fold better on rate, a 2.8-fold better off rate, and a 4.7-fold better affinity than clone g7B07. Finally, the two clones isolated from the CDR2 randomization campaign (20H09 and 23H09) showed distinct binding properties, in line with the screening data. The clone g23H09 showed a similar on rate, a 2.8-fold better affinity (3.9 nM), and a 1.8-fold better off rate compared to clone g7B07. Whereas the clone g20H09 showed a 3-fold higher dissociation rate, but similar affinity. As expected, all the clones showed better affinity and off-rate compared to the engineered variant g7B07_N53A (kd 7.8E-02 1/s, KD 143.5 nM).

**Table 6: Characterization of the binding properties of the selected germline clones on Biacore 3000**

| | | | | | |
|---|---|---|---|---|---|
| Constructs | Rmax (RU) | ka (1/Ms) | kd (1/s) | K_{D} (nM) | kd-fold drop vs g7B07 |
| g18C06 | 815 | 4.1E+05 | 4.8E-04 | 1.2 | 9.8 |
| g23H09 | 1037 | 6.5E+05 | 2.5E-03 | 3.9 | 1.8 |
| g20H09 | 797 | 8.6E+05 | 1.5E-02 | 17.5 | 0.3 |
| g24F02 | 745 | 8.3E+05 | 1.6E-03 | 2.0 | 2.8 |
| g24F02_N53A | 751 | 7.1E+05 | 1.6E-03 | 2.3 | 2.8 |
| g7B07 (mean) | 698 | 4.2E+05 | 4.7E-03 | 11.1 | 1.0 |
| g7B07_N53A (mean) | 178 | 6.4E+05 | 7.8E-02 | 143.5 | 0.06 |

To confirm that the selected clones bind to the 7B07 epitope, an epitope binning analysis of the clones was done, in human Fab format, against 7B07 (human Fab). For this purpose, a sub optimal concentration of the biotinylated clone 7B07 human Fab was added to the Gal10 coated plate pre incubated with the selected clones. The percentage of competition to 7B07 is listed in **Table 7.** Motavizumab (Mota) in human Fab backbone was used as a negative control (0% competition). Clone 7B07 was used as the positive control for competition and was therefore set as the 100% competition value. The anti-human specific clone 1D11 (binding to Tyrosine 69 residue) was included in the tested panel as a negative control for competition with 7B07 for Gal10 binding because it is known that 1D11 binds to the opposite side of Gal10 (including Tyrosine 69) compared to where 7B07 binds. In line with the screening data, all the selected clones were found to compete with 7B07 for Gal10 binding.

**Table 7: Epitope binning of the selected clone anti-Gal10 against 7B07 human Fab biotinylated on ELISA**

| | | |
|---|---|---|
| Clones (hFab) | Mean OD value | % Competition against g7B07-Biot |
| g18C06 | 0.05 | 105.3 |
| g24F02 | 0.26 | 96.5 |
| g24F02_N53A | 0.20 | 99.0 |
| g20H09 | 0.33 | 93.2 |
| g23H09 | 0.31 | 94.3 |
| g7B07 | 0.18 | 100.0 |
| Mota-hFab | 2.51 | 0.0 |
| 1D11-hFab | 1.55 | 41.0 |
| Buffer | 2.37 | 6.0 |

The binding properties of the selected Fab clones to human and cynomolgus Gal10 was analyzed via a capture method established on Biacore 3000. For this purpose, two approaches were used. In the first one, two concentrations of the selected Fab clone were applied on cynomolgus (WGS isoform) Gal10-His capture on a CM5 chip coated with a monoclonal anti-His. In the second approach, a serial dilution was applied on human or cynomolgus (WGS isoform) Gal10 His on the same set up.

In the first phase, the cynomolgus cross reactivity of the clone was tested via the injections of two concentrations on captured cynomolgus Gal10 (WGS isoform). In comparison with previous data generated with the full antibody format, the clone g7B07 and its engineered variant g7B07_N53A showed weak binding to the WGS isoform of the cynomolgus Gal10 (KD 69 nM for g7B07 hFab versus 1.5 nM for g7B07 mlgG1). Among the tested panel, the clone g20H09 showed a poor binding capacity to the cynomolgus antigen, whereas the clone g18C06 showed no binding. However, the clones g23H09, g24F02 and its engineered variant g24F02_N53A showed a good cynomolgus cross reactivity. Further characterization of the cynomolgus cross reactivity of these clones highlighted g24F02_N53A and g23H09. In fact, these two clones showed a 1.4 nM and 5.34 nM affinity on human Gal10 and 8.0 and 9.9 nM affinity on cynomolgus Gal10 respectively. In addition, these two clones (g23H09 and g24F02_N53A) showed a 1.7 fold and 6.3 fold better affinity and 1.9-fold up to 3.5 fold better off rate than clone g7B07. The mutation of the Asn at position 53 in the CDR2 of the g24F02_VH did not translate to reduced binding to human or cynomolgus Gal10, with similar affinity and off rate. In line with the screening data, the clone g18C06 showed the best affinity (1.27 nM) and off-rate (4.9E-04 1/s) of the tested panel on human Gal10, but no binding to its cynomolgus counterpart. Interestingly, the randomization of the 6 amino acids of the CDR2 of the g7B07_VH resulting in the generation of g23H09 showed an increase in cynomolgus cross reactivity compared to the parental clone. However, the clone g20H09, isolated from a similar discovery campaign from which 3 amino acids of the CDR2 had been randomized does not show this gain of cynomolgus cross reactivity, demonstrating that key amino acids have been introduced in the CDR2 of clone g23H09, leading to an increased binding to cynomolgus.

**Table 8: Characterization of the binding properties of the selected clones in human Fab backbone to human and cynomolgus Gal10 (WGS isoform) on Biacore**

| | | | | | | | | |
|---|---|---|---|---|---|---|---|---|
| | Human Gal10-His | | | | Cynomolgus Gal10-His (WGS isoform) | | | |
| Clones (hFab) | ka (1/Ms) | kd (1/s) | Amplitude R0 (RU) 30 nM | K_{D} (M) | ka (1/Ms) | kd (1/s) | Amplitude R0 (RU) 30 nM | K_{D} (M) |
| g7B07 | 6.7E+05 | 6.0E-03 | 607 | 9.08 | 1.9E+06 | 1.3E-01 | 64 | 69.5 |
| g7B07_N53A | 5.4E+05 | 7.9E-02 | 119 | 145.00 | 5.1E+05 | 2.1E-01 | 30 | 417.0 |
| g23H09 | 5.9E+05 | 3.1E-03 | 722 | 5.34 | 9.5E+05 | 9.5E-03 | 589 | 9.9 |
| g18C06 | 3.8E+05 | 4.9E-04 | 598 | 1.27 | no binding | no binding | no binding | no binding |
| g24F02 | 1.1E+06 | 1.6E-03 | 644 | 1.49 | 1.4E+06 | 7.8E-03 | 566 | 5.4 |
| g24F02_N53A | 1.3E+06 | 1.8E-03 | 647 | 1.43 | 1.1E+06 | 8.8E-03 | 544 | 8.0 |

### 3.3 Confirming stability of the Fab clones

To avoid a similar issue as faced with clone 7B07, where after stress testing a deamidation site in the CDR2 of the variable domain of the heavy chain induced a clear drop in binding and potency, the stability of the selected clones was analyzed. For this purpose, a short accelerated temperature stress study was performed. In this set up, stressed samples were incubated for two weeks at 37°C before being analyzed side by side to non-stressed samples (T0) for binding (Biacore was done at argenx) and potency (CLC dissolution was done at VIB).

To analyze the binding properties of the two weeks temperature stressed samples of the selected clones, the captured method established on Biacore 3000 was optimized. Briefly, calibrators and QC points were determined prior to temperature stressing. The binding properties of the stressed samples were determined, compared to the calibration point, and expressed as a percentage of Relative Activity (% RA).

As illustrated in **Table 9,** the clones 18C06 and 24F02_N53A showed the best stability after two weeks at 37°C, with a similar binding capacity as the non-temperature stressed samples (106% and 99% RA, respectively). Besides, the non-engineered variant of 24F02 did not show any loss in binding after two weeks of incubation at 37°C, demonstrating that deamidation at position 53 does not affect its binding property. The clones 23H09 and 20H09 showed a reduced binding capacity after the incubation at 37°C, which resulted in 78% and 86% of RA after two weeks respectively. However, this 14% and 22% drop in binding can either be considered within the assay variation or a need for a longer temperature stress study.

**Table 9: Analysis of the binding properties of the temperature stressed samples of the selected clones**

| | | | |
|---|---|---|---|
| Clones | From | TO | T2W |
| g18C06 | 7B07-epitope like | 108 | 106 |
| g20H09 | CDR2 random (X3) | 110 | 86 |
| g23H09 | CDR2 random (X6) | 98 | 78 |
| g24F02 | Heavy chain shuffling | 101 | 175* |
| g24F02_N53A | Heavy chain shuffling | 98 | 99 |

### 3.4 Confirming potency of the Fab clones

The potency of the temperature stressed samples of the selected clones in human Fab backbone to dissolve recombinant CLC was tested at Charles River Laboratories. The clone 1D11 was included as a reference to correct for inter assay variation. The crystals' dissolution was monitored over time via InCell 2200 after 2, 5, 7, and 16 hours of incubation. The crystal generation carried out before the runs demonstrated different sizes of CLC, varying from 5-10 µm (Run 2) up to 10-20 µm (Run 1). In line with previous observations, the crystal size has a strong impact on the efficacy of the compound and the CLC with a size between 10-20 µm allowed the best discrimination between the clones. The CLC dissolution data generated at Charles River Laboratories showed that the clones 20H09 and 23H09, both isolated from the CDR2 randomization campaign, were the most potent clones in the panel **(Table 10).** Indeed, these clones were capable of dissolving 59.6% and 68.6% of the recombinant CLCs within 2 hours (Run 1). Overall, after 5 hours of incubation, most clones (except 1D11) were able to solubilize ~90% of the CLCs. The positive control, clone 1D11, showed the lowest potency to dissolve the crystals with 20% and 36% CLC dissolution after 2 and 5 hours of incubation respectively.

In line with the other stability results (binding), the temperature stressed samples of clones 18C06, 20H09, and 23H09 showed similar potency as the non-stressed samples.

**Table 10: Characterization of the potency of the temperature stress samples of the selected clones (human Fab) to dissolve recombinant CLC on a cell imaging system at Charles River Laboratories**

| | | | | | | | | |
|---|---|---|---|---|---|---|---|---|
| Range crystal (µm) | 10-20 | | 5-10 | | 10-20 | | 5-10 | |
| | Run 1_2h | | Run 2_2h | | Run 1_5h | | Run 2_5h | |
| Clones | TO | 2W | TO | 2W | TO | 2W | TO | 2W |
| g18C06 | 36.6 | 43.4 | 81.4 | 81.4 | 76.8 | 78.8 | 92.1 | 91.4 |
| g23H09 | 59.6 | 52.9 | 87.9 | 85.6 | 84.1 | 80.6 | 94.0 | 90.9 |
| g20H09 | 68.6 | 70.0 | 91.0 | 86.7 | 91.4 | 88.8 | 93.7 | 93.6 |
| 1D11 | 20.5 | / | 62.1 | / | 36.2 | / | 85.0 | / |

As the clone 24F02_N53A came later in the panel, the potency of this clone to dissolve the recombinant CLC could not be tested at Charles River Laboratories. However, the potency of 24F02_N53A to dissolve recombinant CLC was analyzed with a spinning disk confocal microscope at VIB. For this purpose, the human Fab fragments were incubated with pre formed CLC and dissolution of the crystal was monitored over time. In line with the binding data, the temperature stress sample (2 weeks at 37°C) showed similar efficacy compared to the non-stressed samples **(Table 11),** highlighting the stability of this clone for two weeks at 37°C. In fact, the tested samples from this clone showed a 50% CLC dissolution within 43-46 minutes. Regardless whether or not 24F02 was stressed at 37°C, it showed a close to, but slightly lower, potency as compared to 7B07.

**Table 11: Time lapse of the solubilization of recombinant Charcot Leyden Crystals (CLC) by the temperature stress samples of clone 24F02_N53A (hFab) on a spinning disk confocal microscope**

| | | |
|---|---|---|
| Clones (hFab) | 50% dissolution (min) | 90% dissolution (min) |
| g7B07 | 34.44 | 135.1 |
| g24F02_N53A_T0 | 43.7* | >170 |
| g24F02_N53A_2W | 45.95* | >170 |

| | | |
|---|---|---|
| * Partial curve | | |

### 3.5 Sequences of the Fab clones

| | **g24F02_N53A** | **SEQ ID NO:** |
|---|---|---|
| **VH** | EVQLLESGGGLVQPGGSLRLSCAASGFTFSSYAMSWVRQA PGKGLEWVSAINAGGGSTSYADSVKGRFTISRDNSKNTLYL QMNSLRAEDTAVYYCAKGLRVGVLGFDYWGQGTLVTVSS | 1 |
| **HCDR1** | SYAMS | 2 |
| **HCDR2** | AINAGGGSTSYADSVKG | 3 |
| **HCDR3** | GLRVGVLGFDY | 4 |
| **VL** | SYELTQPLSVSVALGQTARITCDGNNIGSKSAQWYQQKPGQ APVLVIYADEYRPEGIPERFSGSNSGNTATLTISRAQAGDEA DYYCQVWDGSAAVFGGGTQLTVL | 5 |
| **LCDR1** | DGNNIGSKSAQ | 6 |
| **LCDR2** | ADEYRPE | 7 |
| **LCDR3** | QVWDGSAAV | 8 |

| | **g18C06** | **SEQ ID NO:** |
|---|---|---|
| **VH** | EVQLLESGGGLVQPGGSLRLSCAASGFTFSSFAMSWVRQA PGKGLEWVSGINSGDGSSVKGRFTISRDNSKNTLYLQMNSL RAEDTAVYYCAKGGATVWGRFHDYWGQGTLVTVSS | 10 |
| **HCDR1** | SFAMS | 11 |
| **HCDR2** | GINSGDGSSVKG | 12 |
| **HCDR3** | GGATVWGRFHDY | 13 |

| | | |
|---|---|---|
| **VL** | QSVLTQPPSVSGAPGQRVTISCTGSSSNIGSGSYLSWYQQL PGTAPKLLIYNANNRASGVPDRFSGSKSGTSASLAITGLQAE DEADYYCGCYDSKLSTYVFGGGTKLTVL | 14 |
| **LCDR1** | TGSSSNIGSGSYLS | 15 |
| **LCDR2** | NANNRAS | 16 |
| **LCDR3** | GCYDSKLSTYV | 17 |

| | **g20H09** | **SEQ ID NO:** |
|---|---|---|
| **VH** | EVQLLESGGGLVQPGGSLRLSCAASGFTFRSYDMSWVRQA PGKGLEWVSAIKWHRITYYADSVKGRFTISRDNSKNTLYLQM NSLRAEDTAVYYCATTHGIGTLGFGSWGQGTLVTVSS | 18 |
| **HCDR1** | SYDMS | 19 |
| **HCDR2** | AIKWHRITYYADSVKG | 20 |
| **HCDR3** | THGIGTLGFGS | 21 |
| **VL** | SYELTQPLSVSVALGQTARITCDGNNIGSKSAQWYQQKPGQ APVLVIYADEYRPEGIPERFSGSNSGNTATLTISRAQAGDEA DYYCQVWDGSAAVFGGGTQLTVL | 5 |
| **LCDR1** | DGNNIGSKSAQ | 6 |
| **LCDR2** | ADEYRPE | 7 |
| **LCDR3** | QVWDGSAAV | 8 |

| | **g23H09** | **SEQ ID NO:** |
|---|---|---|
| **VH** | EVQLLESGGGLVQPGGSLRLSCAASGFTFRSYDMSWVRQA PGKGLEWVSAIAQFQHWTYYADSVKGRFTISRDNSKNTLYL QMNSLRAEDTAVYYCATTHGIGTLGFGSWGQGTLVTVSS | 22 |
| **HCDR1** | SYDMS | 19 |
| **HCDR2** | AIAQFQHWTYYADSVKG | 23 |
| **HCDR3** | THGIGTLGFGS | 21 |
| **VL** | SYELTQPLSVSVALGQTARITCDGNNIGSKSAQWYQQKPGQ APVLVIYADEYRPEGIPERFSGSNSGNTATLTISRAQAGDEA DYYCQVWDGSAAVFGGGTQLTVL | 5 |
| **LCDR1** | DGNNIGSKSAQ | 6 |
| **LCDR2** | ADEYRPE | 7 |
| **LCDR3** | QVWDGSAAV | 8 |

| | **g24F02** | **SEQ ID NO:** |
|---|---|---|
| **VH** | EVQLLESGGGLVQPGGSLRLSCAASGFTFSSYAMSWVRQA PGKGLEWVSAINNGGGSTSYADSVKGRFTISRDNSKNTLYL QMNSLRAEDTAVYYCAKGLRVGVLGFDYWGQGTLVTVSS | 24 |
| **HCDR1** | SYAMS | 2 |
| **HCDR2** | AINNGGGSTSYADSVKG | 25 |
| **HCDR3** | GLRVGVLGFDY | 4 |
| **VL** | SYELTQPLSVSVALGQTARITCDGNNIGSKSAQWYQQKPGQ APVLVIYADEYRPEGIPERFSGSNSGNTATLTISRAQAGDEA DYYCQVWDGSAAVFGGGTQLTVL | 5 |
| **LCDR1** | DGNNIGSKSAQ | 6 |
| **LCDR2** | ADEYRPE | 7 |
| **LCDR3** | QVWDGSAAV | 8 |

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended clauses. Moreover, all aspects and embodiments of the invention described herein are considered to be broadly applicable and combinable with any and all other consistent embodiments, including those taken from other aspects of the invention (including in isolation) as appropriate.

Various publications and patent applications are cited herein, the disclosures of which are incorporated by reference in their entireties.

### The invention can also be understood with reference to the following numbered clauses:

1. A pharmaceutical composition comprising a Fab fragment that binds to galectin-10, for use in a method of treating a disease or condition associated with the presence or formation of galectin-10 crystals, wherein the method comprises administering to a subject in need thereof a therapeutically effective amount of the pharmaceutical composition, wherein the composition is administered as an aerosol.
2. A method of treating a disease or condition associated with the presence or formation of galectin-10 crystals, wherein the method comprises administering to a subject in need thereof a therapeutically effective amount of a pharmaceutical composition comprising a Fab fragment that binds to galectin-10, wherein the composition is administered as an aerosol.
3. The pharmaceutical composition for use according to clause 1, or the method of clause 2, wherein the disease or condition associated with the presence or formation of galectin-10 crystals is selected from the group consisting of: asthma; chronic rhinosinusitis; celiac disease; helminth infection; gastrointestinal eosinophilic inflammation; cystic fibrosis (CF); allergic bronchopulmonary aspergillosis (ABPA); Churg-Straus vasculitis; chronic eosinophilic pneumonia; and acute myeloid leukemia.
4. The pharmaceutical composition for use or the method of clause 3, wherein the disease or condition associated with the presence or formation of galectin-10 crystals is asthma or cystic fibrosis (CF).
5. The pharmaceutical composition for use according to any one of clauses 1-4, or the method of any one of clauses 2-4, wherein the pharmaceutical composition is administered to the subject via inhalation.
6. The pharmaceutical composition for use according to any one of clauses 1-5, or the method of any one of clauses 2-5, wherein the Fab fragment inhibits crystallization of galectin-10 when bound to soluble galectin-10, and/or promotes dissolution of crystalline galectin-10 when bound to crystalline galectin-10.
7. The pharmaceutical composition for use or the method of clause 6, wherein the crystalline galectin-10 is Charcot-Leyden crystals (CLCs).
8. The pharmaceutical composition for use according to any one of clauses 1-7, or the method of any one of clauses 2-7, wherein the Fab fragment binds to human galectin-10.
9. The pharmaceutical composition for use according to any one of clauses 1-8, or the method of any one of clauses 2-8, wherein the Fab fragment comprises a variable heavy chain (VH) domain and a variable light chain (VL) domain,
   wherein the VH domain comprises:
      HCDR3 comprising or consisting of SEQ ID NO: 4 (GLRVGVLGFDY);
      HCDR2 comprising or consisting of SEQ ID NO: 3 (AINAGGGSTSYADSVKG); and
      HCDR1 comprising or consisting of SEQ ID NO: 2 (SYAMS); and
   wherein the VL domain comprises:
      LCDR3 comprising or consisting of SEQ ID NO: 8 (QVWDGSAAV);
      LCDR2 comprising or consisting of SEQ ID NO: 7 (ADEYRPE); and
      LCDR1 comprising or consisting of SEQ ID NO: 6 (DGNNIGSKSAQ).
10. The pharmaceutical composition for use according to any one of clauses 1-9, or the method of any one of clauses 2-9, wherein the Fab fragment comprises a variable heavy chain (VH) domain and a variable light chain (VL) domain, wherein:
   the VH domain comprises the amino acid sequence of SEQ ID NO: 1 or an amino acid sequence having at least 90%, 95%, 97%, 98% or 99% identity thereto, and
   the VL domain comprises the amino acid sequence of SEQ ID NO: 5, or an amino acid sequence having at least 90%, 95%, 97%, 98% or 99% identity thereto.
11. The pharmaceutical composition for use or the method of clause 10, wherein the Fab fragment comprises a variable heavy chain (VH) domain and a variable light chain (VL), wherein:
   the VH domain comprises the amino acid sequence of SEQ ID NO: 1 
(EVQLLESGGGLVQPGGSLRLSCAASGFTFSSYAMSWVRQAPGKGLEWVSAINAGGG
STSYADSVKGRFTISRDNSKNTLYLQMNSLRAEDTAVYYCAKGLRVGVLGFDYWGQGT
LVTVSS);
   and the VL domain comprises the amino acid sequence of SEQ ID NO: 5 
(SYELTQPLSVSVALGQTARITCDGNNIGSKSAQWYQQKPGQAPVLVIYADEYRPEGIPE
RFSGSNSGNTATLTISRAQAGDEADYYCQVWDGSAAVFGGGTQLTVL).
12. The pharmaceutical composition for use according to any of clams 1-11, or the method of any of clauses 2-11, wherein the Fab fragment is humanized, germlined, or deimmunized.
13. The pharmaceutical composition for use according to any of clams 1-12, or the method of any of clauses 2-12, wherein the particle size of particles in the aerosol is less than 30 µm, less than 25 µm, less than 10 µm, less than 2 µm, less than 1 µm, less than 0.5 µm, less than 0.4 µm, less than 0.3 µm, less than 0.2 µm, or less than 0.1 µm.
14. The pharmaceutical composition for use according to any of clams 1-13, or the method of any of clauses 2-13, wherein the concentration of particles in the aerosol is less than 2 x10⁵, less than 1 x10⁵, less than 2 x 10⁴, or less than 1 x 10⁴ total particles/ml.
15. The pharmaceutical composition for use according to any of clams 1-14, or the method of any of clauses 2-14, wherein the concentration of particles of size greater than 2 µm in the aerosol is less than 1 x10⁵, less than 5 x 10⁴, less than 2 x10⁴, less than 1 x 10⁴, less than 1 x 10³, or less than 1 x 10² particles > 2 µm /ml.
16. The pharmaceutical composition for use according to any of clams 1-15, or the method of any of clauses 2-15, wherein the pharmaceutical composition is administered as an aerosol using a nebulizer, a pressurised metered-dose inhaler (pMDI), a dry powder inhaler, a soft-mist inhaler, or a smart inhaler.
17. The pharmaceutical composition for use or the method of clause 16, wherein the pharmaceutical composition is administered as an aerosol using a nebulizer.
18. The pharmaceutical composition for use or the method of clause 17, wherein the nebulizer is a jet nebulizer, an ultrasonic nebulizer, or a mesh nebulizer.
19. The pharmaceutical composition for use according to any of clams 1-18, or the method of any of clauses 2-18, wherein prior to administration the composition is an aqueous liquid formulation.
20. The pharmaceutical composition for use according to any of clams 1-19, or the method of any of clauses 2-19, wherein the composition comprises a surfactant in an amount less than 5%, less than 2%, less than 1%, less than 0.5%, less than 0.1 %, less than 0.05%, less than 0.02% or less than 0.01%.
21. The pharmaceutical composition for use according to any of clams 1-19, or the method of any of clauses 2-19, wherein the composition does not contain surfactant.
22. The pharmaceutical composition for use according to any of clams 1-21, or the method of any of clauses 2-21, wherein the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml.
23. The pharmaceutical composition for use according to any of clams 1-21, or the method of any of clauses 2-21, wherein the concentration of the Fab fragment in the composition is about 1, 2, 3, 4, 5, 6, 7, 8, 9, 10, 11, 12, 13, 14 or 15 mg/ml.
24. The pharmaceutical composition for use according to any of clams 1-23, or the method of any of clauses 2-23, wherein the composition comprises a pharmaceutically acceptable carrier or excipient.
25. The pharmaceutical composition for use or the method of clause 24, wherein the excipient is a liquid excipient, optionally a solvent or an aqueous solvent.
26. A pharmaceutical composition comprising a Fab fragment that binds to galectin-10, wherein the composition is formulated for aerosolization.
27. The pharmaceutical composition of clause 26, wherein the composition is an aqueous liquid formulation.
28. The pharmaceutical composition of clause 26 or clause 27, wherein the composition comprises a surfactant in an amount less than 5%, less than 2%, less than 1%, less than 0.5%, less than 0.1%, less than 0.05%, less than 0.02% or less than 0.01%.
29. The pharmaceutical composition of clause 26 or clause 27, wherein the composition does not contain surfactant.
30. The pharmaceutical composition of any one of clauses 26-29, wherein the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml.
31. The pharmaceutical composition of any one of clauses 26-29, wherein the concentration of the Fab fragment in the composition is about 1, 2, 3, 4, 5, 6, 7, 8, 9, 10, 11, 12, 13, 14 or 15 mg/ml.
32. The pharmaceutical composition of any one of clauses 26-31, wherein the composition comprises a pharmaceutically acceptable carrier or excipient.
33. The pharmaceutical composition of clause 32, wherein the excipient is a liquid excipient, optionally a solvent or an aqueous solvent.
34. The pharmaceutical composition of any one of clauses 26-33, wherein the Fab fragment comprises a variable heavy chain (VH) domain and a variable light chain (VL) domain,
   wherein the VH domain comprises:
      HCDR3 comprising or consisting of SEQ ID NO: 4 (GLRVGVLGFDY);
      HCDR2 comprising or consisting of SEQ ID NO: 3 (AINAGGGSTSYADSVKG); and
      HCDR1 comprising or consisting of SEQ ID NO: 2 (SYAMS); and
   wherein the VL domain comprises:
      LCDR3 comprising or consisting of SEQ ID NO: 8 (QVWDGSAAVF);
      LCDR2 comprising or consisting of SEQ ID NO: 7 (ADEYRPE); and
      LCDR1 comprising or consisting of SEQ ID NO: 6 (DGNNIGSKSAQ).
35. A nebulizer comprising the pharmaceutical composition of any one of clauses 26-34.

## Claims

1. A pharmaceutical composition comprising a Fab fragment that binds to galectin-10, for use in a method of treating a disease or condition associated with the presence or formation of galectin-10 crystals, wherein the method comprises administering to a subject in need thereof a therapeutically effective amount of the pharmaceutical composition, wherein the composition is administered as an aerosol, wherein the disease or condition associated with the presence or formation of galectin-10 crystals is selected from the group consisting of: asthma; chronic rhinosinusitis; celiac disease; helminth infection; gastrointestinal eosinophilic inflammation; cystic fibrosis (CF); allergic bronchopulmonary aspergillosis (ABPA); Churg-Straus vasculitis; chronic eosinophilic pneumonia; and acute myeloid leukemia.

2. The pharmaceutical composition for use according to claim 1, wherein:
(i) the pharmaceutical composition is administered to the subject via inhalation;
(ii) the Fab fragment inhibits crystallization of galectin-10 when bound to soluble galectin-10, and/or promotes dissolution of crystalline galectin-10 when bound to crystalline galectin-10, optionally wherein the crystalline galectin-10 is Charcot-Leyden crystals (CLCs); and/or
(iii) the Fab fragment binds to human galectin-10.

3. The pharmaceutical composition for use according to claim 1 or claim 2, wherein the Fab fragment comprises a variable heavy chain (VH) domain and a variable light chain (VL) domain,
wherein the VH domain comprises:
HCDR3 comprising or consisting of SEQ ID NO: 4 (GLRVGVLGFDY);
HCDR2 comprising or consisting of SEQ ID NO: 3 (AINAGGGSTSYADSVKG); and
HCDR1 comprising or consisting of SEQ ID NO: 2 (SYAMS); and
wherein the VL domain comprises:
LCDR3 comprising or consisting of SEQ ID NO: 8 (QVWDGSAAV);
LCDR2 comprising or consisting of SEQ ID NO: 7 (ADEYRPE); and
LCDR1 comprising or consisting of SEQ ID NO: 6 (DGNNIGSKSAQ).

4. The pharmaceutical composition for use according to any one of claims 1-3, wherein the Fab fragment comprises a variable heavy chain (VH) domain and a variable light chain (VL) domain, wherein:
the VH domain comprises the amino acid sequence of SEQ ID NO: 1 or an amino acid sequence having at least 90%, 95%, 97%, 98% or 99% identity thereto, and
the VL domain comprises the amino acid sequence of SEQ ID NO: 5, or an amino acid sequence having at least 90%, 95%, 97%, 98% or 99% identity thereto.

5. The pharmaceutical composition for use according to any of clams 1-4, wherein:
(i) the particle size of particles in the aerosol is less than 30 µm, less than 25 µm, less than 10 µm, less than 2 µm, less than 1 µm, less than 0.5 µm, less than 0.4 µm, less than 0.3 µm, less than 0.2 µm, or less than 0.1 µm;
(ii) the concentration of particles in the aerosol is less than 2 x10⁵, less than 1 x10⁵, less than 2 x 10⁴, or less than 1 x 10⁴ total particles/ml; and/or
(iii) the concentration of particles of size greater than 2 µm in the aerosol is less than 1 x10⁵, less than 5 x 10⁴, less than 2 x10⁴, less than 1 x 10⁴, less than 1 x 10³, or less than 1 x 10² particles > 2 µm /ml.

6. The pharmaceutical composition for use according to any of clams 1-5,
wherein the pharmaceutical composition is administered as an aerosol using a nebulizer, a pressurised metered-dose inhaler (pMDI), a dry powder inhaler, a soft-mist inhaler, or a smart inhaler; optionally wherein the pharmaceutical composition is administered as an aerosol using a nebulizer; further optionally wherein the nebulizer is a jet nebulizer, an ultrasonic nebulizer, or a mesh nebulizer.

7. The pharmaceutical composition for use according to any of clams 1-6, wherein prior to administration the composition is an aqueous liquid formulation; optionally wherein the composition comprises a surfactant in an amount less than 5%, less than 2%, less than 1%, less than 0.5%, less than 0.1%, less than 0.05%, less than 0.02% or less than 0.01%, or wherein the composition does not contain surfactant.

8. The pharmaceutical composition for use according to any of clams 1-7, wherein
(i) the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml; or
(ii) the concentration of the Fab fragment in the composition is about 1, 2, 3, 4, 5, 6, 7, 8, 9, 10, 11, 12, 13, 14 or 15 mg/ml.

9. The pharmaceutical composition for use according to any of clams 1-8, wherein the composition comprises a pharmaceutically acceptable carrier or excipient, optionally wherein the excipient is a liquid excipient, optionally a solvent or an aqueous solvent.

10. A pharmaceutical composition comprising a Fab fragment that binds to galectin-10, wherein the composition is formulated for aerosolization.

11. The pharmaceutical composition of claim 10, wherein the composition is an aqueous liquid formulation, optionally wherein the composition comprises a surfactant in an amount less than 5%, less than 2%, less than 1%, less than 0.5%, less than 0.1 %, less than 0.05%, less than 0.02% or less than 0.01%, or wherein the composition does not contain surfactant.

12. The pharmaceutical composition of claim 10 or 11, wherein
(i) the concentration of the Fab fragment in the composition is between about 1 and about 20 mg/ml, between about 1 and about 15 mg/ml, between about 1 and about 10 mg/ml, between about 1 and about 5 mg/ml, between about 2 and about 10 mg/ml, between about 3 and about 10 mg/ml, between about 4 and about 10 mg/ml, or between about 5 and about 10 mg/ml; or
(ii) the concentration of the Fab fragment in the composition is about 1, 2, 3, 4, 5, 6, 7, 8, 9, 10, 11, 12, 13, 14 or 15 mg/ml.

13. The pharmaceutical composition of any one of claims 10-12, wherein the composition comprises a pharmaceutically acceptable carrier or excipient, optionally wherein the excipient is a liquid excipient, optionally a solvent or an aqueous solvent.

14. The pharmaceutical composition of any one of claims 10-13, wherein the Fab fragment comprises a variable heavy chain (VH) domain and a variable light chain (VL) domain,
wherein the VH domain comprises:
HCDR3 comprising or consisting of SEQ ID NO: 4 (GLRVGVLGFDY);
HCDR2 comprising or consisting of SEQ ID NO: 3 (AINAGGGSTSYADSVKG); and
HCDR1 comprising or consisting of SEQ ID NO: 2 (SYAMS); and
wherein the VL domain comprises:
LCDR3 comprising or consisting of SEQ ID NO: 8 (QVWDGSAAV);
LCDR2 comprising or consisting of SEQ ID NO: 7 (ADEYRPE); and
LCDR1 comprising or consisting of SEQ ID NO: 6 (DGNNIGSKSAQ).

15. A nebulizer comprising the pharmaceutical composition of any one of claims 10-14.
